# EUROPEAN PATENT SPECIFICATION

(11) **EP 1 563 100 B1**
(45) Date of publication and mention of the grant of the patent: **24.04.2013**
(21) Application number: 03810829.6
(22) Date of filing: 03.11.2003
(51) Int. Cl.: C12Q 1/68, G01N 33/53, G01N 33/536, G01N 33/537, G01N 33/543

(54) **DISPLACEMENT SANDWICH IMMUNO-PCR**
VERDRÄNGUNGS-SANDWICH-IMMUNO-PCR
IMMUNO-PCR SANDWICH PAR DEPLACEMENT

(30) Priority: 01.11.2002 US 423173 P
(43) Date of publication of application: 17.08.2005
(60) Divisional application: 10184768.9
(73) Proprietor: Iris Molecular Diagnostics, Inc., Chatsworth CA 91311 (US)
(72) Inventor: JABLONSKI, Ed, Carlsbad, CA 92011 (US); DRIVER, David, Carlsbad, CA 92011 (US); ADAMS, Tom, Carlsbad, CA 92011 (US)
(74) Representative: Viering, Jentschura & Partner
(86) International application number: PCT/US2003/035153
(87) International publication number: WO 2004/042030

(56) References cited:
- EP-A- 1 249 500
- WO-A-92/16841
- WO-A-02/083951
- WO-A2-02/068695
- US-A- 5 702 896
- US-A- 5 985 548
- US-B1- 6 531 278
- NIEMEYER CHRISTOF M ET AL: "DNA-directed immobilization: Efficient, reversible, and site-selective surface binding of proteins by means of covalent DNA-streptavidin conjugates" ANALYTICAL BIOCHEMISTRY, ACADEMIC PRESS, SAN DIEGO, CA, US, vol. 268, no. 1, 1 March 1999 (1999-03-01), pages 54-63, XP002176566 ISSN: 0003-2697
- JABLONSKI E ET AL: "Preparation of oligodeoxynucleotide-alkaline phosphatase conjugates and their use as hybridization probes.", NUCLEIC ACIDS RESEARCH 11 AUG 1986 LNKD- PUBMED:3748805, vol. 14, no. 15, 11 August 1986 (1986-08-11), pages 6115-6128, ISSN: 0305-1048

## Description

### FIELD OF THE INVENTION

This invention relates to methods for generating a signal in response to an analyte in present in a sample. Specifically, the invention relates to methods that employ detection by signal amplification of a nucleic acid marker. This invention also relates to a methods for labeling antibodies or other proteins with a nucleic acid, especially DNA; methods for purifying an antibodies and analytes; and methods for screening antibodies for diagnostic and therapeutic applications.

### BACKGROUND OF THE INVENTION

One typical immunoassay is known as Enzyme Linked Immunosorbent Assay (ELISA). The antigen to be detected is removed from solution by non-specific adsorbtion to a solid surface, such as the inside of a microtiter plate well. After washing to remove excess antigen and blocking the remaining sites on the plate, the immobilized antigen is contacted with a primary antibody to form an immune complex on the solid support. Excess antibody can be removed by washing the plate. The primary antibody is detected via an enzyme, which can be directly conjugated to the primary antibody (direct ELISA) or to a second anti-antibody, which is subsequently contacted with the plate (indirect ELISA). The enzyme activity associated with the solid surface is proportional to the amount of bound antigen present and can be measured, for example, by using a chromogenic substrate for the enzyme.

Immuno-PCR is similar to indirect ELISA, but uses a DNA sequence in place of an enzyme as the detection molecule (*see, e.g.,* Sano et al., Science, 1992, 258:120-22). Sano *et al.,* demonstrated the efficacy of this method with a bovine serum albumin (BSA) antigen and anti-BSA primary antibody. Biotinylated plasmid DNA was coupled to the primary antibody via protein A-avidin. Amplification of the antigen-associated DNA by 30 cycles of polymerase chain reaction (PCR) permitted detestation by staining with ethidium bromide following gel electrophoresis. The authors reported an enhanced detection sensitivity of approximately five orders of magnitude when compared to ELISA detection. Theoretically, a DNA label may be detected with extraordinary sensitivity (potentially down to a single copy) when amplified by PCR or any other exponential nucleic acid amplification technique. However, the sequential addition of each assay component requires extensive washing to sufficiently reduce non-specifically bound material.

Immuno-PCR can also be performed in a sandwich immunoassay format. A typical sandwich immunoassay employs two antibodies recognizing the antigen to be detected. A "capture" antibody is used to coat the surface of a solid support, such as a microtiter well, bead or particle. A "reporter" antibody is labeled with a detection molecule, such as a radioactive isotope, a fluorophore or an enzyme. In some cases the same antibody can be used for both capture and reporter. Typically, the antigen is first contacted with either the reporter antibody in solution or with the capture antibody on the solid support, followed by addition of the remaining antibody. The resulting specific immune complex is bound to the surface of the solid support and consists of the antigen combined with two antibodies. Excess antigen and antibodies are removed by repeatedly washing the support. It is important to maintain the integrity of the immobilized immune complex during washing steps to maximize the signal strength while enhancing the removal of the unbound reporter antibody, thereby minimizing background. Following the washes, the bound reporter antibody is measured via the detection molecule as an indication of the amount of antigen present.

The reporter antibody may be labeled directly by covalent modification of the antibody or indirectly through the binding of a second antibody or protein possessing a detectable label. Indirect attachment may be accomplished, for example, by labeling the reporter antibody with biotin and attaching the detection molecule to avidin. When the detection system is sufficiently sensitive, the assay's lower limit will usually be determined by the degree of background produced by nonspecific binding of unbound reporter antibody.

Examples of sandwich immuno-PCR have also been reported in the literature (*see, e.g.,* Joerger et al., Clin Chem, 1995, 41:1371-77; Hendrickson et al., Nucleic Acids Res, 1995, 23:522-529). Detection limits for the sandwich Immuno-PCR assay format exceed those of conventional enzyme immunoassay. However, stringent washing, for example using detergent solution, is required to remove non-specifically bound DNA-labeled reporter antibodies.

EP 1 249 500 relates to a method for the detection of an analyte, wherein a solid support is coupled with a first nucleic acid sequence and a complementary second sequence is coupled to an antibody by means of biotin-avidin interaction using a biotinylated nucleic acid sequence and an antibody coupled to avidin. Also discloses is the coupling of a PCR-detectable marker nucleic acid to a second analyte-specific antibody by means of biotin-avidin interactions.

The sandwich format of Immuno-PCR has also been used to demonstrate the detection of multiple antigens ("multiplexing") (*see e.g.,* U.S. Pat. No. 5,985,548). DNA labels of different length were each coupled to reporter antibodies recognizing distinct antigens using a 5' N-hydroxysuccinimide ester (NHS). The reporter antibody-DNA conjugates bound to a solid support coated with the various antigens. The multiple DNA labels were detected simultaneously in the assay based on the sizes of the respective PCR amplification products, which had been designed to allow resolution from each other by gel electrophoresis.

Immuno-PCR offers a real improvement in sensitivity over present immunoassays, but is plagued by significant opportunities for generating background. Background can be generated from nonspecific binding of any component, including the reporter antibody-DNA conjugate, the chimeric protein, or other secondary labeling component. Since single molecules of nucleic acid can be detected by PCR, failure to remove all of the nonspecifically bound template DNA molecules results in significant background and interferes with the ability to detect minute quantities of analyse. Stringent, numerous or prolonged washes to reduce non-specific have been used to reduce background, but can result in reduced signal due to elution or dissociation of antigen/antibody complexes. Furthermore, Immuno-PCR is limited by the well-known non-linearity of PCR, which can frustrate attempts to assess the proportionate amounts of analyte present These practical difficulties have prevented Immuno-PCR techniques from gaining widespread acceptance and utility in the field.

A variety of methods have been suggested to reduce background in both standard immunoassays and Immuno-PCR methods. Ishikawa et al. (U.S. Pat. No. 5,888,834) has proposed the technique of immune complex transfer immunoassay, in which a sandwich immune complex is released from the capturing support and re-captured onto a fresh surface. Non-specifically bound reporter components are left behind on the initial solid support. WO 02/83951 discloses the binding of an analyte by a conjugate of an analyte-binding ligand, a PNA sequence and a label, such as biotin. Unbound conjugate is removed by binding to a solid phase or by passing through a membrane. The PNA-label is then preferably released from the analyte-conjugate complex by trypsin-mediated cleavage of the PNA from the ligand. The PNA-label is then captured on a solid support via interactions between the PNA and a DNA sequence coupled to the solid support, with subsequent detection of the label. Nucleic acid hybridization assay sensitivity has also been improved by chemically eluting hybridized DNA from a solid support, leaving behind nonspecifically bound reporter DNA probe (*see* Morrissey, et al., Anal Biochem 1989, 181:345-359).

Target DNA was hybridized with dA-tailed capture probe and a labeled reporter probe. *Id* The complex was captured onto paramagnetic beads coated with oligo dT. *Id*. After washing, the complex was released by eluting with a solution of guanidinium thiocyanate and recaptured on a fresh support coated with poly dT. *Id* Complexes have also been released by the use of homopolymers, such as 200-400 by poly (rU), in combination with washing in tetraalkylammonium salts (Collins et al., US Patent No. 5,702,896).

Conceptually, Immuno-PCR has extraordinary theoretical sensitivity. The technique, however, has not been exploited commercially due to the problem of nonspecifically bound template DNA label, which obscures the true analyte signal. This leads to the generation of false positive results and irreproducibility when attempting to detect analytes present at low levels. Some investigators have concluded that immuno-PCR is no more sensitive than ELISA in detecting IgG due to nonspecific binding (McKie, et al., J. Immunol. Meth., 2002, 261:167175). The remarkable sensitivity achieved by Sano *et al.* was demonstrated with a pure system using a cumbersome, multi-step format, impractical for medical applications.

There is a need in the field of diagnostics and biosciences for a robust assay to detect analytes present at vanishingly low levels or concentrations. This need will be more keenly felt following the sequencing and analysis of the human genome. This massive sequencing effort has resulted in the discovery of previously unrecognized proteins which must be analyzed and characterized. Many of these proteins may be present in amounts undetectable by current technology. The promise of Immuno-PCR to provide highly sensitive methods for analyte detection has only been partially fulfilled. Thus, there is a need to provide methods for immuno-PCR that have high sensitivity and low background.

### SUMMARY OF THE INVENTION

The invention relates to a process by which a capture antibody for a sandwich immunoassay is labeled with a specific double-stranded DNA molecule where the strand complementary to that attached to the capture antibody is modified with a high affinity ligand. Use of the ligand allows the complementary DNA strand to be attached to a solid support. The capture antibody/double-stranded DNA is then immobilized onto a solid phase comprising the corresponding high affinity ligand binding protein with the nucleic acid bridge linking the capture antibody to the solid phase. In preferred embodiments, a biotin/avidin binding pair is used, but other combinations of ligands and ligand-binders may also be employed. The solid phase may serve as a medium to capture antigen or antigen-reporter antibody immune complexes. The DNA strand may be imparted with properties allowing for the release of the immune complex from the capture medium to enhance detection sensitivity.

Although the instant invention is described herein by the exemplary use of DNA molecules as a bridge, displacer nucleic acids, and marker nucleic acids, in other embodiments nucleic acids other than DNA may be used, such as RNA or peptide nucleic acid (PNA), for one strand, both strands, or portions of one or both strands.

Diagnostic kits for performing such methods may be constructed to include a first container containing the capture antibody linked to a solid support with a nucleic acid bridge; a second container having a conjugate of the same or a different antibody and a marker DNA molecule; and a third container comprising a displacer nucleic acid. The diagnostic kit may also include notification of a FDA approved use and instructions therefor. One skilled in the art will readily recognize that antibodies other than those described in the present invention can readily be incorporated into one of the established kit formats that are well known in the art.

This invention also relates to the process of labeling antibodies (or other proteins) with a DNA molecule of predetermined sequence. The DNA molecule can be subsequently detected with great sensitivity by PCR or any nucleic acid amplification technique. Alternatively, the DNA molecule can be used to bridge the antibody or protein to a solid support The DNA molecule may be attached directly to the antibody by first chemically activating the DNA, and then allowing the DNA molecule to bind to the antibody. A ligand binding protein may also be labeled with chemically-activated DNA, and then allowed to form a complex with the reporter antibody derivatized with the corresponding ligand for the binding protein. Thus, either the reporter antibody, the capture antibody, or another protein may be attached to a DNA molecule via a direct covalent attachment.

Kits for performing such methods may be constructed to include a container having disposed therein a nucleic acid of predetermined sequence activated to be ready to bind to a selected protein.

In one aspect, a kit for performing nucleic acid detection immunoassays comprises activated DNA molecules for labeling reporter and/or capture antibodies where the reporter antibody is to be labeled with a DNA marker strand and the capture antibody is to be labeled with a strand of a nucleic acid bridge. The kit further comprises strands complimentary to the above-mentioned DNA molecules where the DNA complimentary in whole or in part to the above-mentioned DNA strand from the nucleic acid bridge is biotinylated, and a displacer strand that is complementary in whole or in part to the above-mentioned DNA strand from the nucleic acid bridge but is not biotinylated. The kit may further comprise amplification primer(s), avidinylated paramagnetic micro particles, binding and wash buffers, PCR reagents, and a dye for detecting the amplification product, such as SYBR Green.

### BRIEF DESCRIPTION OF THE DRAWINGS

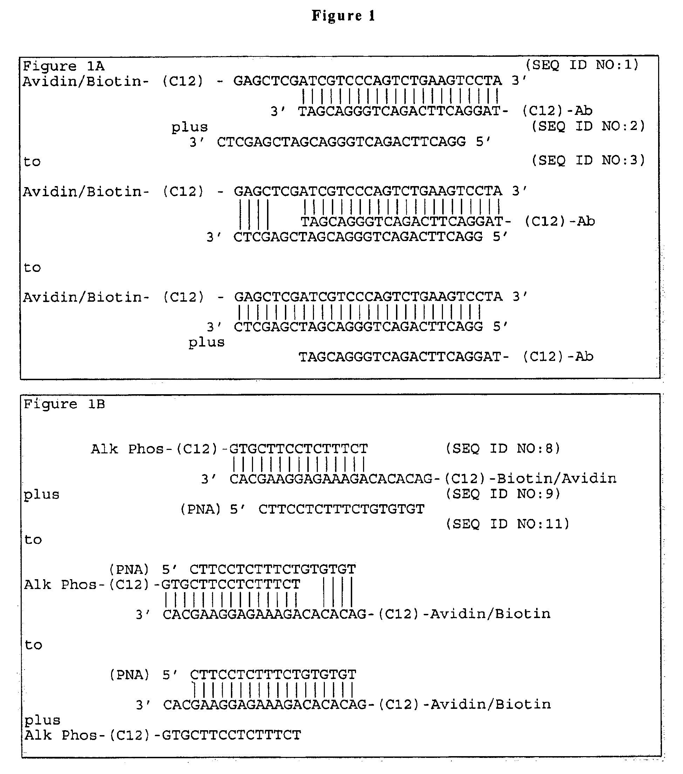

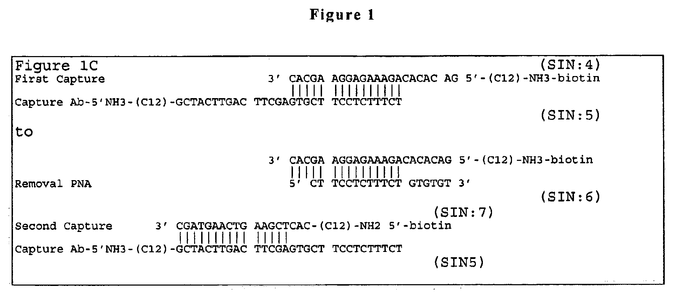

**Figure 1****:** Figure 1A depicts a DNA sequence, attached to antibody, and hybridized to the sequence anchored to the solid support via a 22 base hybridized region. The displacer molecule hybridizes to anchor sequence at the solid support end via a 7 base GC-rich footing and proceeds to displace the capture sequence along the next 20 bases, leaving a AT hybrid at the antibody attachment end, which is unstable. Figure 1B depicts an experiment where, in place of a capture antibody, alkaline phosphatase is conjugated to a nucleic acid molecule and then bridged to a solid support. Figure 1C depicts a first capture, followed by a displacement, followed by a recapture.
**Figure 2****:** Background signal obtained by the displacement of the immune complex from the paramagnetic microparticles.
**Figure 3****:** Stability of Capture/Anchor Hybrid. Figure 3 shows that the capture/anchor hybrid is stable under the salt conditions of the buffer used. The antibody/biotinylated DNA conjugate remains bound to the well, as well as controls.
**Figure 4****:** PNA Displacement and Salt Concentration. Figure 4 shows the effect of PNA displacement on the bound antibody/biotinylated DNA conjugate. At low ionic strength, PNA displacement is very effective, removing the antibody within a few minutes. It is not as effective at high salt.
**Figure 5****:** DNA Displacement and Salt Concentration. Figure 5 shows displacement by DNA. It is very effective at high salt, and ineffective in the absence of salt.
**Figure 6:** Figure 6 shows the result of a real-time PCR experiment. Figure 6A shows the real-time fluorescence signal obtained from a standard dilution series of anti-PSA rMAb-DNA conjugates. Figure 6B shows the threshold cycle as a function of the initial number of rMAb-DNA molecules from (A).

### DETAILED DESCRIPTION OF THE INVENTION

### Definitions

The term "**analyte**," as used herein, refers to any substance that can be detected in an assay, and which may be present in a sample. The analyte can be, without limitation, any substance and preferably comprises a substance for which there exists a naturally occurring antibody or for which an antibody can be prepared. The analyte may, for example, be a protein, a polypeptide, a hapten, a carbohydrate, a lipid, a cell or any other of a wide variety of biological or non-biological molecules, complexes or combinations thereof. In preferred embodiments of the invention, analytes a do not contain nucleic acids.

The term "**non-nucleic acid analyte**" refers generally to an analyte the does not contain a nucleic acid that will interfere with immuno-PCR detection methods of the invention. Non-nucleic acid analytes of the invention do not solely consist of a nucleic acid molecules and typically, the non-nucleic acid analyte does not contain nucleic acids. In preferred embodiments, the analyte may comprise a peptide, a protein, a carbohydrate, a lipid, a cell or any antigenic material. Analytes of the invention may comprise a nucleic acid component, but the binding of the analyte to be detected is not dependent on complementary hybridization between any nucleic acid sequence in the analyte and a nucleic acid sequence in the receptor provided by the invention.

The term "**sample**" as used herein refers to an aliquot of material, frequently an aqueous solution or an aqueous suspension derived from biological material. Samples to be assayed for the presence of an analyte by the methods of the present invention include, for example, cells, tissues, homogenates, lysates, extracts, and purified or partially purified proteins and other biological molecules and mixtures thereof. Nonlimiting examples of samples typically used in the methods of the invention include human and animal body fluids such as whole blood, serum, plasma, cerebrospinal fluid, sputum, bronchial washing, bronchial aspirates, urine, lymph fluids and various external secretions of the respiratory, intestinal and genitourinary tracts, tears, saliva, milk, white blood cells, myelomas and the like; biological fluids such as cell culture supernatants; tissue specimens which may or may not be fixed; and cell specimens which may or may not be fixed. The samples used in the methods of the present invention will vary based on the assay format and the nature of the tissues, cells, extracts or other materials, especially biological materials, to be assayed. Methods for preparing protein extracts from cells or samples are well known in the art and can be readily adapted in order to obtain a sample that is compatible with the methods of the invention.

The term "**contacting**" as used herein refers generally to providing access of one component, reagent, analyte or sample to another. For example, contacting can involve mixing a solution comprising a non-nucleic acid receptor with a sample. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples. In one embodiment of the invention, contacting involves adding a solution comprising a non-nucleic acid receptor to a sample utilizing a delivery apparatus, such as a pipette-based device or syringe-based device.

The terms "**non-nucleic acid receptor**" or "**receptor**" as used herein refer to a non-nucleic acid molecule that is capable of specifically binding another molecule. In one embodiment, the receptor is an antibody. In a preferred aspect of this embodiment, the receptor is a reporter antibody. In other embodiments of the invention, receptors can include, without limitation: biological receptor molecules, such as the insulin receptor; ligands for receptors, such as insulin; and other biological molecules that have affinity for another molecule, such as biotin and avidin. The receptors of the present invention need not comprise an entire naturally occurring molecule but may only consist of a portion, fragment or subunit of a naturally occurring molecule, as for example the Fab fragment of an antibody. Receptors may be generated by any method known in the art. For example, antibodies may be found in a serum, prepared from a hybridoma tissue culture supernatant or ascites fluid, or may be derived from a recombinant expression system, as will be well known in the art. Fragments, portions or subunits of *e.g*., an antibody or other receptor, may be generated by chemical, enzymatic or other means, yielding for example, well-known (*e.g*., Fab, Fab') or novel molecules. The present invention also contemplates that receptors can include recombinant, chimeric and hybrid molecule, such as humanized and primatized antibodies, and other non-naturally occurring antibody forms. Those skilled in the art will recognized that the non-limiting examples given above describing various forms of antibodies can also be extended to other receptors such that recombinant, chimeric, hybrid, truncated etc., forms of non-antibody receptors can be used in the methods of the present invention.

"**Polyclonal antibodies**" are heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

"**Monoclonal antibodies**," which are homogeneous populations of antibodies to a particular antigen, may be obtained by any technique that provides for the production of antibody molecules by continuous cell lines in culture. These include, but are not limited to the hybridoma technique of Kohler and Milstein, Nature, 256:495-7 (1975); and U.S. Patent No. 4,376,110), the human B-cell hybridoma technique (Kosbor, et al., Immunology Today, 4:72 (1983); Cote, et al., Proc. Natl. Acad. Sci. USA, 80:2026-30 (1983)), and the EBV-hybridoma technique (Cole, et al., in Monoclonal Antibodies And Cancer Therapy, Alan R. Liss, Inc., New York, pp. 77-96 (1985)). Such antibodies may be of any immunoglobulin class including IgG, IgM, IgE, IgA, IgD and any subclass thereof. The hybridoma producing the mAb of this invention maybe cultivated *in vitro* or *in vivo.* Production of high titers of mAbs *in vivo* makes this apresently preferred method of production.

In addition, techniques developed for the production of "**chimeric antibodies**" (Morrison, et al., Proc. Natl. Acad. Sci., 81:6851-6855 (1984); Takeda, et al., Nature, 314:452-54 (1985)) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies (U.S. Patent No. 4,946,778; Bird, Science 242:423-26 (1988); Huston, et al., Proc. Natl. Acad. Sci. USA, 85:5879-83 (1988); and Ward, et al., Nature, 334:544-46 (1989)) can be adapted to produce gene-single chain antibodies. Single chain antibodies are typically formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F(ab')₂ fragments that can be produced by pepsin digestion of the antibody molecule and the Fab fragments that can be generated by reducing the disulfide bridges of the F(ab')₂ fragments. Alternatively, Fab expression libraries may be constructed (Huse, et al., Science, 246:1275-81 (1989)) to allow rapid and easy identification of monoclonal Fab fragments with the desired specificity.

By the terms "**specifically binding**" and "**specific binding**" as used herein is meant that an antibody or other molecule, especially a receptor of the invention, binds to a target such as an antigen, ligand or analyte, with greater affinity than it binds to other molecules under the specified conditions of the present invention. Antibodies or antibody fragments, as known in the art, are polypeptide molecules that contain regions that can bind other molecules, such as antigens. In various embodiments of the invention, "specifically binding" may mean that an antibody or other biological molecule, binds to a target molecule with at least about a 10⁶-fold greater affinity, preferably at least about a 10⁷-fold greater affinity, more preferably at least about a 10⁸-fold greater affinity, and most preferably at least about a 10⁹-fold greater affinity than it binds molecules unrelated to the target molecule. Typically, specific binding refers to affinities in the range of about 10⁶-fold to about 10⁹-fold greater than non-specific binding. In some embodiments, specific binding may be characterized by affinities greater than 10⁹-fold over non-specific binding.

The terms "**polynucleotide**" and "**nucleic acid (molecule)**" are used interchangeably to refer to polymeric forms of nucleotides of any length. The polynucleotides may contain deoxyribonucleotides, ribonucleotides and/or their analogs. Nucleotides may have any three-dimensional structure, and may perform any function, known or unknown. The term "**polynucleotide**" includes single-, double-stranded and triple helical molecules. "**Oligonucleotide**" refers to polynucleotides of between 5 and about 100 nucleotides of single- or double-stranded nucleic acid, typically DNA. Oligonucleotides are also known as oligomers or oligos and may be isolated from genes, or chemically synthesized by methods known in the art. A "**primer**" refers to an oligonucleotide, usually single-stranded, that provides a 3'-hydroxyl end for the initiation of enzyme-mediated nucleic acid synthesis. The following are non-limiting embodiments of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A nucleic acid molecule may also comprise modified nucleic acid molecules, such as methylated nucleic acid molecules and nucleic acid molecule analogs. Analogs of purines and pyrimidines are known in the art, and include, but are not limited to, aziridinycytosine, 4-acetylcytosine, 5-fluorouracil, 5-bromouracil, 5-carboxymethylaminomethyl-2-thiouracil, 5-carboxymethyl-aminomethyluracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, pseudouracil, 5-pentylnyluracil and 2,6-diaminopurine. The use of uracil as a substitute for thymine in a deoxyribonucleic acid is also considered an analogous form of pyrimidine.

The term "**nucleic acid marker**" refers to a nucleic acid molecule that will produce a detection product of a predicted size or other selected characteristic when used with appropriately designed oligonucleotide primers in a nucleic acid amplification reaction, such as a PCR reaction. Skilled artisans will be familiar with the design of suitable oligonucleotide primers for PCR and programs are available over the Internet to facilitate this aspect of the invention (*see,* for example, the http site: bibiserv.techfak.unibielefeld.de/ genefisher). A nucleic acid marker may be linear or circular. In preferred embodiments, the nucleic acid marker will comprise a predetermined, linear nucleic acid sequence with binding sites for selected primers located at or near each end. In a circular DNA nucleic acid molecule, the primers will be internal rather than at an end, and a single primer may be used, *e.g*. for Rolling Circle Amplification. Amplified DNA may be detected using any available method, including, but not limited to techniques such as real time PCR, SYBR Green staining, or ethidium bromide staining. In other embodiments of the invention, the binding sites for the amplification primers flank an undefined DNA sequence of defined length or which comprises another identifiable characteristic, such as a detectable sequence, in addition to undefined sequences. In some embodiments, the nucleic acid marker are distinguished by the size or mass of the amplified sequences; thus, the DNA sequence between the primers need not be defined as to the exact sequence, just as to the number of bases. Alternatively, the size and/or sequence of the entire nucleic acid marker need not be defined as long as a binding site for a molecular beacon (*see*, *infra*) is supplied. In further embodiments, the DNA sequence located between the primer binding sites comprises a "characteristic identification sequence" capable of being detected during the PCR reaction. Fluorescent signal generation may, for example, be sequence-specific (Molecular Beacons, Taq Man, fluorogenic primers, such as the LUX primers (Invitrogen (Carlsbad, CA.)) or mass dependent (SYBR Green, Ethidium Bromide). The examples provided are not meant to be an exhaustive list of possible nucleic acid detection schemes as those skilled in the art will be aware of alternative markers suitable for use in the methods of the present invention.

The term "**characteristic identification sequence**" refers to a nucleic acid sequence that can be specifically detected by virtue of hybridization to oligonucleotide or other nucleic acid that has been labeled with a detectable marker such as a radioisotope, a dye (such as a fluorescent dye), or other species that will be known in the art. In preferred embodiments, the characteristic identification sequence is capable of binding a "molecular beacon" probe. The term "**molecular beacon**" refers to oligonucleotides such as those sold by Operon Technologies (Alameda, CA) and Synthetic Genetics (San Diego, CA). (*See* also, Tyagi and Kramer, Nat Biotechnol, 1996, 14:303-308; and Tyagi et al., Nat Biotechnol, 2000, 18:1191-96.). As noted herein, a signal can be generated using a variety of techniques and reagents.

The term "**analyte-dependent reporter complex**" refers to the complex formed by a receptor that has been labeled with a nucleic acid marker and bound to a non-nucleic acid analyte. In a preferred embodiment, the complex is specific, stable and has a high degree of specificity.

The term "**solid support**" refers any solid phase that can be used to immobilize e.g., an analyte, an antibody or a complex. Suitable solid supports will be well known in the art and include the walls of wells of a reaction tray, such as a microtiter plate, the walls of test tubes, polystyrene beads, paramagnetic or non-magnetic beads, nitrocellulose membranes, nylon membranes, microparticles such as latex particles, and sheep (or other animal) red blood cells. Typical materials for solid supports include, but are not limited to, polyvinyl chloride (PVC), polystytrene, cellulose, nylon, latex and derivatives thereof. Further, the solid support may be coated, derivatized or otherwise modified to promote adhesion of the desired molecules (*e.g*., analytes) and/or to deter non-specific binding or other undesired interactions. The choice of a specific "solid phase" is usually not critical and can be selected by one skilled in the art depending on the assay employed. Thus, latex particles, microparticles, paramagnetic or non-magnetic beads, membranes, plastic tubes, walls of microtiter wells, glass or silicon chips, and red blood cells are all suitable sold supports. Conveniently, the solid support can be selected to accommodate various detection methods. For example, 96 or 384 well plates can be used for assays that will be automated, for example by robotic workstations, and/or those that will be detected using, for example, a plate reader. For methods of the present invention that may involve an autoradiographic or chemiluminescent detection step utilizing a film-based visualization, the solid support may be a thin membrane, such as a nitrocellulose or nylon membrane. According to one embodiment of the invention in which sandwich immunoassays are performed, the walls of the wells of a reaction tray are typically employed. In alternative embodiments of the instant invention, paramagnetic beads may be used as a solid support. Suitable methods for immobilizing molecules on solid phases include ionic, hydrophobic, covalent interactions and the like, and combinations thereof. However, the method of immobilization is not typically important, and may involve uncharacterized adsorbtion mechanisms. A "solid support" as used herein, may thus refer to any material which is insoluble, or can be made insoluble by a subsequent reaction. The solid support can be chosen for its intrinsic ability to attract and immobilize a capture reagent. Alternatively, the solid phase can retain an additional receptor which has the ability to attract and immobilize a capture reagent. The additional receptor may include a substance that is oppositely charged with respect to either the capture reagent itself or to a charged substance conjugated to the capture reagent. In yet another embodiment of the invention, an additional receptor molecule can be any specific binding member which is immobilized upon (attached to) the solid phase and which has the ability to immobilize a capture reagent through a specific binding reaction. The additional receptor molecule enables indirect immobilization of the capture reagent to a solid phase before or during the performance of the assay. The solid phase thus can be a plastic, derivatized plastic, paramagnetic or non-magnetic metal, glass or silicon surface of a test tube, microtiter well, sheet, bead, microparticle, chip, or other configurations known to those of ordinary skill in the art.

The term "**nucleic acid bridge**" refers to a partially or wholly double-stranded nucleic acid moiety that functions to connect non-nucleic acid receptors of the invention to a solid support. The nucleic acid bridge typically consists of two nucleic acid molecules, one of which is attached to a solid support of the invention, while the other is attached to the non-nucleic acid receptor. These two nucleic acid molecules hybridize to each other by their complementary regions, thereby connecting the non-nucleic acid receptor to the solid support. In some embodiments of the invention, DNA is used as the nucleic acid bridge. In other embodiments, nucleic acids other than DNA may be used, such as RNA or peptide nucleic acid (PNA), for one strand, both strands, or portions of one or both strands. In preferred embodiments of the present invention, the double-stranded portion of the nucleic acid bridge is completely complementary. However, in other embodiments, the double-stranded portion of the nucleic acid bridge is substantially complementary. By the term "**substantially complementary**" is meant that two nucleic acid sequences have at least about 80% or more nucleic acid complementarity, preferably at least about 90% or more nucleic acid complementarity, more preferably at least about 95% or more nucleic acid complementarity, and most preferably at least about 99% or more nucleic acid complementarity. Complementarity describes the ability of nucleotide bases in one nucleic acid strand to pair with an opposing base in another nucleic acid strand. As is well known in the art, adenine bases in DNA pair with thymidine, while cytosine and guanine form a complementary pair. Complementarity is measured by dividing the number of complementary bases pairs in two nucleic acid sequences by the total number of bases and multiplying the product by 100. Thus, exact complements display 100% complementarity along their entire length, while sequences that are less similar and have, *e.g*., mismatched bases, have a lower degree of complementarity. In typical embodiments of the invention, the complementarity is sufficient to maintain double-stranded binding of the nucleic acid bridge prior to the use of the displacer nucleic acid, under the conditions employed.

In preferred embodiments, the strand of the nucleic acid bridge attached to the non-nucleic acid receptor is attached covalently by a linking group at its 5' end. In preferred embodiments, the strand of the nucleic acid bridge attached to the solid support is attached thereto by a biotin/avidin bridge wherein the biotin is covalently attached to the 5' end of this nucleic acid bridge molecule, and the biotin binds to a biotin-receptor, such as avidin, attached to the solid support. In preferred embodiments, the nucleic acid bridge attached to the solid support is covalently attached to the solid support by a linking group at the 5' end of the nucleic acid molecule. In further preferred embodiments, the sequence of the nucleic acid molecule attached to the solid support is selected from the group consisting of SEQ ID NO:1, SEQ ID NO:4, and SEQ ID NO:7. In other embodiments, the partially complementary strand that is attached to a capture non-nucleic acid receptor (or reporter enzyme such as alkaline phosphatase) is selected from the group consisting of SEQ ID NO:2, and SEQ ID NO:5. In additional embodiments, the non-nucleic acid receptor is an antibody. These embodiments are exemplary only, and other nucleic acid sequences may be used, as will be apparent to one of skill in the art. Furthermore, other means may be used to attach the nucleic acid bridge to the solid support.

The term "**detecting**" as used herein refers to any method of verifying the presence of a given molecule. The techniques used to accomplish this may include, but are not limited to, PCR, sequencing, PCR sequencing, molecular beacon technology, hybridization, and hybridization followed by PCR. Examples of reagents which might be used for detection include, but are not limited to, radiolabeled probes, enzymatic labeled probes (horseradish peroxidase, alkaline phosphatase), and affinity labeled probes (biotin, avidin, or streptavidin).

The term "**eluted**" or "**eluting**" refers to extracting, dissociating or removing a bound or adsorbed material from an adsorbent by means of a molecule, buffer, or solvent in a controlled fashion. In one embodiment, the elution uses a selected molecule to disrupt, dissociate or destabilize the link to the solid support without significantly changing the buffer or other conditions. In one aspect of this embodiment, the selected molecule competes with the bound or adsorbed material for binding to the adsorbent In a preferred embodiment, the selected molecule is a nucleic acid molecule that comprises a region of sequence complementarity to the nucleic acid strand of a double-stranded nucleic acid bridge attached to the solid support. In still other embodiments, the elution can be accomplished using restriction enzymes or endonucleases which are well known in the art. Examples of common restriction enzymes include, but are not limited to as Eco RI, Hind III and Nco L

The term "**displacer nucleic acid**" refers to a nucleic acid of predetermined sequence that is used to elute an analyte-dependent reporter complex from a solid support. In preferred embodiments of the invention, the sequence is selected from the group consisting of SEQ ID NO:3, and SEQ ID NO:6.

In one embodiment of the invention, the displacer nucleic acid is a DNA molecule. In other embodiments, the displacer nucleic acid is an RNA molecule. In further embodiments, the displacer nucleic acid is a PNA molecule. In still other embodiments, the displacer nucleic acid is a phosphorothioate molecule.

The term "**nucleic acid replication composition**" refers to compositions that provide a means of replicating a nucleic acid sequence. Typically, the composition will consist of such components as buffer, salts, co-factors, enzymes, primers and/or other reagents required for replicating a nucleic acid. Replication can be by any means available for replicating the target nucleic acid sequence and the skilled artisan will recognize that a wide variety of methods are available. In preferred embodiments of the invention, the means of replicating DNA is the polymerase chain reaction (*see, e.g.,* Sambrook and Russell, Molecular Cloning, a Laboratory Manual, 3rd ed., 2001). In other nonlimiting embodiments of the invention, amplification is performed by the techniques of (1) isothermal amplification of DNA (*see e.g.,* PCT Interantional Application No. WO 00/41524); (2) Single Temperature Strand Displacement Amplification (SDA) (see e.g., U.S. Pat. No. 5,270,184); (3) Transcription Mediated Amplification (TMA) (U.S. Pat. No. 5,766,849); or Ligase Chain Reaction (LCR) (*see e.g.,* Wu & Wallace, 1989, Genomics 4:560).

The term "**hapten**" as used herein, refers to a small proteinaceous or non-protein antigenic determinant which is capable of being recognized by an antibody. Typically, haptens do not elicit antibody formation in an animal unless part of a larger species. For example, small peptide haptens are frequently coupled to a carrier protein such as keyhole limpet hemocyanin in order to generate an anti-hapten antibody response. **"Antigens"** are macromolecules capable of generating an antibody response in an animal and being recognized by the resulting antibody. Both antigens and haptens comprise at least one antigenic determinant or "**epitope**," which is the region of the antigen or hapten which binds to the antibody. Typically, the epitope on a hapten is the entire molecule.

**"Peptide"** generally refers to a short chain of amino acids linked by peptide bonds. Typically peptides comprise amino acid chains of about 2-100, more typically about 4-50, and most commonly about 6-20 amino acids. "**Polypeptide**" generally refers to individual straight or branched chain sequences of amino acids that are typically longer than peptides. **"Polypeptides"** usually comprise at least about 100 to 1000 amino acids in length, more typically at least about 150 to 600 amino acids, and frequently at least about 200 to about 500 amino acids. **"Proteins"** include single polypeptides as well as complexes of multiple polypeptide chains, which may be the same or different Multiple chains in a protein may be characterized by secondary, tertiary and quaternary structure as well as the primary amino acid sequence structure, may be held together, for example, by disulfide bonds, and may include post-synthetic modifications such as, without limitation, glycosylation, phosphorylation, truncations or other processing. Antibodies such as IgG proteins, for example, are typically comprised of four polypeptide chains (*i.e*., two heavy and two light chains) that are held together by disulfide bonds. Furthermore, proteins may include additional components such associated metals (*e.g*., iron, copper and sulfur), or other moieties. The definitions of peptides, polypeptides and proteins includes, without limitation, biologically active and inactive forms; denatured and native forms; as well as variant, modified, truncated, hybrid, and chimeric forms thereof. The peptides, polypeptides and proteins of the present invention may be derived from any source or by any method, including, but not limited to extraction from naturally occurring tissues or other materials; recombinant production in host organisms such as bacteria, fungi, plant, insect or animal cells; and chemical synthesis using methods that will be well known to the skilled artisan.

As used herein, the term "distinguishable" refers to an ability to distinguish experimentally between the two different markers or species. In embodiments in which an assay is used to detect multiple analytes, none of the multiple DNA marker molecules will consist of sequences identical in both length and sequence. Two markers may comprise the same core sequence, but the markers will be distinguishable on the basis of size and/or different sequences. In preferred embodiments, the PCR products will be different in length. In other embodiments, the markers will have sequences which bind to different sequence specific probes.

The term **"conjugate"** as used herein refers to two molecules which have been covalently attached, or otherwise linked together. In one embodiment, a nucleic acid conjugate is generated by covalently linking the nucleic acid to a polypeptide. In a preferred embodiment of the invention, a non-nucleic acid receptor, as used herein, and a polypeptide are covalently attached via a linking group to form a conjugate.

A **"kit"** for detecting the presence of a selected analyte in a sample by the methods of the invention comprises at least one container means having disposed therein the capture antibody linked to the solid support of the kit by a nucleic acid bridge. The kit may also comprise a container means having disposed therein the reporter antibody, or a container means having disposed therein the displacer nucleic acid. The kit may further comprise other containers comprising one or more of the following: wash reagents, reagents capable of amplifying the bound nucleic acid probe reagents, and agents capable of detecting the presence of bound nucleic acid probe after amplification. Preferably, the kit further comprises instructions for use. The kit, if intended for diagnostic use, may also include notification of a FDA approved use and instructions therefor.

Specifically, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris-buffers, and the like), and containers which contain the reagents used to detect the marker nucleic acid, amplified product, or the like. One skilled in the art will readily recognize that the antibodies labeled with nucleic acid molecules described in the present invention can readily be incorporated into one of the established kit formats that are well known in the art.

A kit for coupling DNA to an antibody or other protein by the method of the invention comprises at least one container means having disposed therein the lyophilized activated DNA. The kit may further comprise other containers comprising one or more of the following: wash reagents, and agents capable of detecting the presence of attached nucleic acid probe after the reaction. Preferably, the kit further comprises instructions for use. One skilled in the art will readily recognize that the activated nucleic acids described in the present invention can readily be incorporated into one of the established kit formats that are well known in the art.

### Displacement Sandwich Immuno-PCR

The present invention provides methods for detecting analytes of interest in various fields, including clinical diagnostics and biological sciences. The Displacement Sandwich Immuno-PCR method of the present uses a reporter antibody directly labeled with 1 or 2 molecules of oligonucleotide using protein crosslinking chemistry. The 1 or 2 oligonucleotides attached to the reporter antibody may be single- or double-stranded. The invention further relates to the process of detecting an antigen (Ag) in a sample using one or more labeled antibodies. The invention also relates to a process by which a capture antibody or a reporter antibody, or other protein, is labeled with a specific DNA sequence.

According to one method of the invention, a non-nucleic acid analyte present in a sample is detected by:
(i) contacting a sample comprising a non-nucleic acid analyte with a reporter conjugate, wherein the reporter conjugate comprises a first receptor capable of specifically binding the analyte, and a nucleic acid marker;
(ii) allowing the binding of the analyte to the reporter conjugate, thereby forming an analyte-dependent reporter complex;
(iii) contacting the analyte-dependent reporter complex with a second receptor for the analyte, wherein the second receptor is attached to a solid support via a nucleic acid bridge of predetermined sequence consisting of two at least partially complementary nucleic acid strands one of which is attached to the solid support;
(iv) allowing the binding of the second receptor to the analyte-dependent reporter complex, thereby forming an analyte-dependent reporter/second receptor complex attached to the solid support;
(v) eluting the analyte-dependent reporter/second receptor complex from the solid support with a displacer nucleic acid having a sequence at least partially complementary to one of the nucleic acid bridge strands, and
(vi) specifically detecting the presence of the nucleic acid marker in the eluted analyte-dependent reporter/second receptor complex, wherein the detection of nucleic acid marker indicates the presence of the analyte in the sample.

One aspect of this method is a process commonly known as Immuno-PCR, whereby an antibody (Ab), particularly a monoclonal antibody (MAb), is labeled with a specific sequence of nucleic acid which is typically DNA, for the purpose of detecting the formation of an immune complex by monitoring the enzymatic amplification of, for example, the DNA. The immune complex may be formed with a single DNA-labeled reporter monoclonal antibody (rMAb), or in a sandwich immunoassay format in concert with a capture monoclonal antibody (cMAb). Polyclonal antibodies may also be employed when appropriate. The nucleic acid label may be detected with extraordinary sensitivity, down to a single copy of the molecule, when subjected to amplification by the Polymerase Chain Reaction (PCR), or any other exponential nucleic acid amplification technique (*e.g*., (1) Tabor et al. (WO 00/41524); (2) Single Temperature Strand Displacement Amplification (SDA) (U.S. Pat. No. 5,270,184); (3) Transcription Mediated Amplification (TMA) (U.S. Patent No. 5,766,849; or Ligase Chain Reaction (LCR) (Wu, D.Y. & R.B. Wallace, 1989, Genomics 4, 560).

Typically, the nucleic amplification will be accomplished by PCR using a polymerase. Suitable polymerases are well known in the art and may include without limitation, DNA polymerases, RNA polymerases, transcriptases, and Q-beta polymerases. The skilled artisan will recognize that the choice of polymerase will depend on what type of nucleic acid is amplified. According to one embodiment of the invention, a replication composition comprising reagents and primers for conducting a polymerase chain reaction is used for nucleic acid amplification. The composition will generally comprise the buffer, nucleotides, salts, enzyme and other components required for performing PCR or another amplification procedure.

Methods of the present invention can also be performed using real time PCR, in which a specific sequence of, *e.g*., DNA is quantified by monitoring its rate of amplification during thermocycling in the presence of Taq polymerase, appropriate primers and nucleotide triphosphates. (For a brief discussion of real time PCR, *see* Walker, 2002, Sci 296:557-58.)

Conveniently, the methods of the present invention can be used to detect multiple analytes present in a single sample (*i.e*., multiplexing). For example, a multiplicity of different non-nucleic acid analytes present in a single sample can be simultaneously detecting, by performing the steps of:
(i) contacting a sample comprising a multiplicity of different non-nucleic acid analytes with a multiplicity of reporter conjugates, where each reporter conjugate comprises a first receptor capable of specifically binding one of the multiplicity of non-nucleic acid analytes in the sample, and a nucleic acid marker, and where each reporter conjugate binds specifically to a different analyte and comprises a nucleic acid that is distinguishable from the other nucleic acids in the multiplicity of reporter conjugates;
(ii) allowing the binding of the multiplicity of non-nucleic acid analytes in the sample to the multiplicity of receptor conjugates, thereby forming a multiplicity of analyte-dependent reporter complexes;
(iii) contacting the multiplicity of analyte-dependent reporter complexes with a multiplicity of second receptors, wherein the multiplicity of second receptors is collectively capable of binding to the multiplicity of analytes in the sample, and wherein the second receptors are attached to a solid support via a nucleic acid bridge of predetermined sequence;
(iv) allowing the binding of the second receptors to the analyte-dependent reporter complexes, thereby forming a multiplicity of analyte-dependent reporter/second receptor complexes attached to the solid support;
(v) eluting the multiplicity of analyte-dependent reporter/second receptor complexes from the solid support with at least one displacer nucleic acid; and
(vi) specifically detecting the presence the nucleic acid markers in the eluted multiplicity of analyte-dependent reporter/second receptor complexes by visualizing the replicated nucleic acid marker by ethidium bromide staining., wherein the detection of each distinguishable nucleic acid marker indicates the presence of the corresponding analyte in the sample.

A typical assay according to this method might employ four or more different receptors, such as antibodies, each specific for a distinct analyte. Where antibodies are used as the receptors, the analytes are antigens or haptens, such as serum components. It may be desirable, for example, to simultaneously measure two or more such serum components as hormones, enzymes and disease markers in the same reaction. Thus, one could perform an assay designed to measure risk for diseases by determining the serum concentration of, *e.g*., cholesterol, insulin, PSA, CA125 and other disease related species in blood. According to this aspect of the invention, nucleic acid markers are designed such that each can be distinguished, for example, on the basis of size. Thus, for an assay of four analytes, the nucleic acid markers might be 50, 70, 90 and 110 nucleotides in length, respectively. Following amplification, PCR products can be resolved and quantified using such techniques as gel electrophoresis and mass spectroscopy.

Another aspect of the invention relates to the screening of antibodies for diagnostic and therapeutic application. More specifically, the invention provides methods for labeling test antibodies with a specific sequence of nucleic acid. The nucleic acid label may then be detected with great sensitivity by PCR or any nucleic acid amplification technique. A DNA label, for example, may be attached directly to the antibody using chemically activated DNA. Alternatively, a binding protein may be labeled with chemically-activated DNA and contacted with a reporter antibody derivatized with the corresponding ligand for the binding protein, to form a complex.

The present invention also provides methods for preparing DNA activated with a chemical crosslinking compound and storing the activated DNA it in kit form. According to this method, an oligonucleotide (typically about 10-100 bases) to be attached to an antibody, peptide, polypeptide or protein is synthesized to contain a 5' amino linkage and is activated with excess suberate. The intermediate is quickly isolated and added to a concentrated solution of, *e.g*., monoclonal antibody (MAb). Alternatively, activated oligonucleotide may be stored for future use. The oligonucleotide-MAb conjugate can be purified by gel filtration and anion exchange chromatography. A complimentary strand may be hybridized to the first (e.g., oligo-Mab conjugate), forming a double-strand DNA bridge for use in Displacement Sandwich Immuno-PCR assays (described below). In the case of a capture antibody, the second complimentary strand may be biotinylated with a commercial reagent at the 5' end and used to coat paramagnetic particles or other material containing bound avidin. The solid phase serves as a medium to capture antigen (Ag) or Ag-MAb immune complexes. A DNA strand attached to the capture antibody may be imparted with properties allowing for the release of the immune complex from the capture medium in order to enhance detection sensitivity.

Kits for carrying out the methods of the present invention for labeling proteins, polypeptides or peptides typically comprise a container containing DNA molecules activated with disuccinimidyl suberate. The DNA molecules can, for example, be a first strand of a nucleic acid bridge, or a first strand of a market DNA molecule. The kits may also contain directions for labeling proteins, polypeptides or peptides using the kit.

The present invention also provides novel methods for detecting analytes using nucleic acid displacement. According to these methods, generally, a non-nucleic acid analyte present in a sample is contacted with a reporter conjugate. The reporter conjugate comprises: a first receptor capable of specifically binding the analyte; and a nucleic acid marker. The analyte is then allowed to bind to the reporter conjugate, thereby forming an analyte-dependent reporter complex. The analyte-dependent reporter complex is then contacted with a second receptor for the analyte, wherein the second receptor is attached to a solid support via a nucleic acid bridge of predetermined sequence. Binding of the second reporter to the analyte-dependent reporter complex, forms an analyte-dependent reporter/second reporter complex attached to the solid support, which can then be eluted, for example, using displacer nucleic acid. Finally, the presence of the nucleic acid marker can be detected as an indicator of the presence analyte in the sample.

One application of this embodiment is in Displacement Sandwich Immuno-PCR assay (DSI-PCR). According to this embodiment, excess reporter antibody/DNA conjugate is added in solution to antigen, forming a first immune complex. The complex is captured on a solid support coated with a second antibody via a double-stranded nucleic acid bridge. Extensive washing removes most of the excess reporter antibody (greater than 99%). In preferred embodiments, this analyte/reporter complex is then gently removed by the use of a displacer nucleic acid. The eluted DNA label associated with the immune complex is amplified by PCR, and monitored continuously by the generation of a fluorescent signal. The use of real time PCR, where the rise in the concentration of nucleic acid copies is observed during the course of a PCR amplification reaction, allows the determination of the original number of marker DNA sequences, and thereby the number of analyte molecules. This avoids the uncertainty in quantifying PCR products in reactions carried to completion. A single copy of either marker DNA or the corresponding reporter MAb conjugate can be statistically detected. The presence of ten copies is detected with certainty.

Performing the DSI-PCR assay in the absence of antigen/analyte and without the elution step results in the "detection" of about 5000 copies of reporter MAb-DNA due to nonspecific binding. Further washing does not diminish this background. It has been noted that removal of excess reporter MAb-DNA by extensive washing under a given set of buffer conditions will always leave some reporter nonspecifically bound to the support. Additional washing steps under another set of conditions, such as elevated temperature or ionic strength, would produce a fresh "bloom" of eluted background signal, demonstrating that the prior washing conditions had not been sufficient to remove all background signal.

The difficulty in eliminating nonspecifically bound reporter MAb-DNA suggested eluting or removing the antigen/immune complex (for example, PSA/anti-PSA) from the solid support prior (e.g., avidin paramagnetic particle) to PCR amplification. One means to accomplish this was by interposing a 26 base pair double-stranded spacer arm containing restriction end nuclease sites between the capture MAb and the biotin used to link to the solid support. Complimentary 26-mer DNA molecules containing a 5' amino linkage were synthesized to contain nuclease sites for both Hind III and Eco RI when hybridized. The amino function on one DNA strand was derivatized with biotin. The second strand was conjugated to capture MAb and then hybridized to the biotinylated strand. The double-stranded capture MAb conjugate was fully bound by immobilized avidin and released with both Hind III and, more efficiently, with Eco RI digestion. A captured PSA immune complex was effectively cleaved from the solid support by Eco RI treatment 37° C for 15 min. These conditions, however, also eluted nonspecific reporter Mob-DNA. Sensitivity when using anti-PSA antibody improved to only 5,000,000 copies of PSA. Similar results were obtained with streptavidin- or avidin-coated microtiter plates.

The further reduction of background, and enhancement of assay sensitivity, requires the removal of the specific immune complex from the solid support by the use of a displacer nucleic acid as described in the instant invention. The MAb-Ag-MAb immune complex is released back into solution and transported to a fresh vessel. Nonspecifically bound or trapped reporter MAb is left behind on the solid support or the walls of the original assay container. This has the effect of purifying the specific immune complex. If the release and transfer of the complex is efficient, signal intensity is maintained while background signal is reduced.

The sensitivity of the DSI-PCR assay has been enhanced in this invention by: (1) directly labeling the reporter MAb with a short synthetic strand of marker DNA; (2) employing MAb sandwich pairs with affinity constants > 10¹⁰; (3) reducing the input reporter MAb concentration 1000-fold (to circa 0.01 no); (4) optimizing blocking and washing protocols; and (5) minimizing solid support surface area and capture MAb coating.

In the present invention, further improvements to the DSI-PCR sensitivity have been accomplished by design of a linkage between the capture MAb and the immunoassay support allowing for the specific displacement of immune complex into solution using extremely mild elution conditions specifically tailored to the nucleic acid bridge used.

In one embodiment of the invention, a capture MAb is labeled with a strand of synthetic DNA (capture strand) through a 5' amino linkage. A synthetic DNA strand (anchor strand) containing 5' biotin and a complimentary 3' sequence is hybridized to the capture strand. The capture/anchor-DNA/DNA conjugate is used to coat the surface of avidin microtiter plates or paramagnetic microparticles or another suitable solid support. The capture MAb is subsequently displaced from the support by addition of excess displacer oligonucleotide containing sequences complimentary to both the 5' end of the anchor strand and the 3'end of the capture strand. The displacer hybridizes near the 5' end of the anchor stand and displaces the capture strand as that strand hybridizes near the 3' end of the capture strand. The capture DNA-DNA conjugate is released into solution as a function of the equilibrium and relative stability of the original versus newly hybridized anchor strand.

Displacement is accomplished without alteration in most solution conditions, including, without limitation, pH, temperature, or ionic strength, all of which have been shown to independently release nonspecifically bound reporter MAb-marker DNA conjugate back into solution. Displacer DNA removes the entire MAb-Ag-MAb immune complex and unbound MAb. Only the immune complex contains marker DNA recognized by PCR primers. The concomitant release of excess capture MAb affects neither signal nor background. The application of immune complex displacement to Immuno-PCR results in a 10-fold improvement in detection by background signal reduction. Detection sensitivities of a few hundred molecules of protein antigen (approximately five attograms) have been achieved.

Immuno-PCR, as described by Sano and Cantor, employes an antibody labeled with a biotinylated fragment of DNA via a chimeric protein bridge consisting of avidin and protein A. The DNA label was amplified by the polymerase chain reaction and detected semi-quantitatively by gel electrophoresis followed by staining with ethidium bromide. *Id.* The multi-step complexity of the assay of Sano & Cantor required long incubation and wash cycles, in excess of the half-life of the primary immune complex, resulting in loss of signal and accuracy due to macromolecular dissociation. Although more recent investigators have added improvements, temporal constraints, reagent complexity and DNA template contamination have inhibited a widespread application of this technology. For example, Watanabe and co-workers have embarked on a series of studies to optimize the DNA concentration and streptavidin concentrations for use in conjunction with specific antibodies (Sugawara, et al., Clint Chem. Act, 299:45-54, 2000 (angiotensinogen), Saito et al., Clin Chem., 45:5:665-669, 1999 (tumor necrosis factor-alpha), and Furuya et al., J. Immunol. Methods, 238:173-180, 2000 (interleukin-18)). Joerger *et al.* used DNA conjugated directly to antibodies specific for human thyroid-stimulating hormone and chorionic gonadotropin in preparation for developing an assay capable of detecting both antigens simultaneously (Joerger et al., Clin Chem 41/9:1371-1377, 1995). Subsequently, Hendrickson *et al.* demonstrated a multianalyte immunoassay for simultaneous detection of three analytes (hTSH, hCG and beta-Gal) using DNA-labaled antibodies (Hendrickson et al. Nucl. Acids Res. 14:6115-28, 1986) *see also,* U.S. Pat. No. 5,985,548)). Jablonski *et al.* also described techniques for directly attaching DNA molecules to protein molecules (Jablonski et al., Hucl. Acids Res. 23/3:522-29, 1995). For further methods of conjugating DNA molecules to polypeptides, *see* Hermanson, Bioconjugate Techniques, pp 639-666, 1996.

According to another embodiment, the displacement methods of the present invention purification of antibodies, analytes and complexes thereof can be performer One aspect of this embodiment comprises a method for reversibly binding an antibody/analyte complex to a solid support, the method comprising the steps of:
(i) providing an antibody/analyte complex, where the 5' end of a first nucleic acid is conjugated to the antibody, the analyte comprises an antigen recognized by the antibody, and the analyte is specifically bound to the antibody through the antigen combining site of the antibody;
(ii) providing a solid support comprising a second nucleic acid, wherein the 5'end of the second nucleic acid is bound to the solid support, wherein the second nucleic acid is substantially complementary to the first nucleic acid over its 3' terminus;
(iii) binding the antibody/analyte complex to the solid support by means of a double-stranded nucleic acid bridge, wherein the nucleic acid bridge is formed by hybridization of the unbound ends of the first and second nucleic acids; and
(iv) eluting the antibody/analyte complex from the solid support by means of a displacer nucleic acid.

As described above, methods for eluting the non-nucleic acid analyte-dependent reporter/second receptor complex typically utilize a displacer nucleic acid molecule under conditions that favor displacement of the nucleic acid bridge with the displacer nucleic acid, but reduce or eliminate elution of non-specifically bound species. In alternative embodiments, any means of specifically eluting the non-nucleic acid analyte-dependent reporter/second receptor complex without contaminating non-specific species can be used. For example, an endonuclease, such as a restriction enzyme can be use to digest the double-stranded bridge, thereby eluting the non-nucleic acid analyte-dependent reporter/second receptor complex

In further embodiments of the invention, a method for labeling antibodies or ligand binding proteins directly with DNA and a means for performing nucleic acid detection immunoassays with real-time PCR in a kit format that will be robust in use are provided. The format of the invention allows for the improved and practical application of an Immuno-PCR assay. The methods of the present invention reduce the problem of DNA-marker non-specific binding, which plagues all exponential nucleic acid amplification assays. The present invention also provides for accuracy and quantification by employing real-time PCR detection.

### Kit for Labeling Proteins with Nucleic Acid Molecules

Another aspect of the present invention relates to methods for labeling antibody molecules. According to this aspect, a DNA reagent is provided that, when contacted in solution under appropriate conditions, forms a covalent linkage to, e.g. an antibody. The DNA reagent may be single- or double-stranded. If single-stranded, the DNA label may be converted to a duplex by hybridization with a fully or partially complementary strand.

The DNA reagent is formed by the synthesis of a DNA sequence containing a primary amine functional group. The primary amine may be located anywhere on the DNA molecule, but in preferred embodiments, the amine is located at the 5' terminus of the DNA. The amine-containing DNA is contacted with an excess of amine-reactive cross-linking agent under appropriate reaction conditions and the amine moiety on the DNA reacts with the cross-linking agent The activated DNA is isolated by gel filtration chromatography under conditions that maintain residual cross-linking activity. The DNA reagent is distributed in known quantities into appropriate vessels, quick-frozen and lyophilized to dryness. Antibody (in solution) added to the lyophilizate reacts with the unreacted end of the cross-linking agent to form a covalent conjugate to DNA. Excess hydrolyzed reagent may be removed, for example, by one or more passages through an ultra filter having an appropriate molecular weight cutoff to discriminate antibody from DNA.

The invention further provides methods for labeling a ligand binding protein. A solution of binding protein is used to hydrate the lyophilized DNA reagent (prepared as described above). The unreacted portion of the cross-linking agent combines with specific amino acid residues within the protein structure to form a covalent attachment to the DNA. The DNA-labeled binding protein may be contacted with antibody labeled with the corresponding ligand, resulting in a DNA-antibody conjugate.

A additional aspect of the present invention provides a method for performing the DSI-PCR assay using the DNA-antibody conjugation process. One such immunoassay according to this aspect of the invention utilizes a sandwich format, whereby an immune complex is formed using two antibodies to different epitopes on the same antigen. One of the pair of antibodies (reporter) is labeled with marker DNA. The second antibody (capture) is immobilized on a solid surface to capture the immune complex formed by the reporter antibody and antigen. The antibody-antigen-antibody complex may be formed by combining all reactants simultaneously or sequentially. The immobilized immune complex is separated from excess reporter antibody-DNA conjugate by washing, followed by selective elution, and the marker DNA is detected by PCR amplification.

The PCR reaction is initiated by the addition of the four different nucleoside triphosphates, an oligonucleotide primer pair for each template being amplified, a thermostable enzyme which catalyzes the polymerization of the nucleoside triphosphates from the end of the primer, and any other reagents required for the PCR reaction. Amplification of the DNA template strands is accomplished by successive heating and cooling steps.

The PCR reaction may be detected by common DNA quantification techniques, including gel electrophoresis, dot blot hybridization, and autoradiography. Preferably, PCR is monitored in real time using an automated thermocycler and fluorescence signal generation (*see, e.g.,* Walker, 2002, Science 296:557-58). Florescence signal generation may, without limitation, be sequence-specific (Molecular Beacons, Taq Man, fluorogenic primers) or mass dependent (SYBR Green, Ethidium Bromide).

PCR amplification and detection may be carried out on the immune complexes bound to the solid support, or on complexes dissolved back into solution. Advantageously, the release of bound immune complex from a capturing solid surface enhances sensitivity by eliminating non-specifically bound DNA-reporter antibody conjugate.

Another form of nucleic acid detection immunoassay uses an immunostaining format. A thin section of tissue fixed to a solid surface is contacted with antibody that has been-labeled with a DNA marker. If antigen is present, an immune complex will form on the tissue section. The sample is washed free to remove excess antibody-DNA reagent and individual cells maybe isolated by the technique of laser capture. Individual captured cells may then be lysed, causing the release and solubilization of antigen-antibody complexes. The marker DNA label can be quantified during or after amplification.

The present invention may be better understood by the following examples, which are intended only to illustrate the present invention and should in no way be construed as limiting the subject invention.

### EXAMPLES

### Example 1: Activation of 5' Amino DNA.

A 59 base marker DNA sequence: 
5′ NH3-(C12)-TGCGTAGCGA TGACTAGCTG CTGATCGATA TTAGCTAGCA
TCAGCGATCG ATACGAGCA-3′ (SEQ ID NO:13)
 was synthesized to contain a single primary amine group at the 5' end via a 12-carbon spacer arm (Glen Research, Sterling, VA). The 5' amino-DNA (350 µg) was dissolved in 20 µl 10.05 M sodium phosphate buffer, pH 8.0. Disuccidimidyl suberate was dissolved in dry DMSO at a concentration of 12 mg/ml. A 40 µl aliquot was added immediately to the DNA solution and mixed by gentle re-pipetting. After one minute at room temperature, the reaction mixture was loaded onto a 0.5 x 20 cm G-25 (fine) column equilibrated in 5 mM citric acid, adjusted to pH 5.4 with 0.1 M sodium hydroxide, and developed at a flow rate of 0.5 ml/min at room temperature. The first eluted peak was collected directly into an Centricon YM-10 Centrifugal Filter Device (Millipore Corp., Bedford MA) embedded in ice. The suberate-activated template DNA was concentrated by centrifugation at 5000-x g for 30 minutes at 4 °C, to 200-300 µl. The DNA concentration was determined by absorbance at 260 nm. One A₂₆₀ unit (approximately 30 µl) was distributed into micro centrifuge tubes containing 5 µl of 10% mannitol, and immediately frozen in dry ice/acetone bath. The pellets were lyophilized to dryness, sealed under dry argon and stored frozen.

The activity of the lyophilized activated DNA was assessed by reaction with a small amine-containing molecule. Glutathione at 50 mg/ml in 0.1 M sodium phosphate, pH 8.0 was reacted with a stoichiometric amount of N-ethylmalimide (NEM). The pH was re-adjusted to 8.0 with sodium hydroxide. One A₂₆₀ unit of suberate-activated DNA was dissolved in 10 µl ofNEM-glutathione, then diluted 25-fold with water to 4.0 A₂₆₀ units/ml. Conjugation with NEM-glutathione resulted in a single slower migrating band on 15 % polyacrylamide TBE-urea gel electrophoresis compared to unmodified 5' amino DNA. Suberate activated DNA, purified and lyophilized under the described conditions, typically exhibits nearly 100% conjugation to NEM-glutathione, indicating complete activation and maintenance of amine reactivity.

### Example 2: Conjugation of Monoclonal Antibody to DNA.

One hundred microliters monoclonal antibody to PSA (1 mg/ml) (A45110136P from Biospecific, Emeryville, CA) was concentrated and exchanged into 0.1 M sodium phosphate, 0.15 M sodium chloride, pH 8.25 by two passes through a Microcon YM-50 centrifugal filtration device at 10,000 x g in a microcentrifuge at room temperature. The final volume of 25 to 50 µl was collected by centrifugation into a microcentrifuge tube and added to a lyophilized pellet of activated marker DNA. The conjugation reaction was allowed to proceed for several hours at room temperature.

The degree of antibody labeling was assessed by SDS and native protein gel electrophoresis. The incorporation of one or more strands of DNA into an antibody structure increases both the molecular weight and negative charge density. Antibodies labeled with one or more strands of DNA appear as distinct slower migrating bands on 4% SDS gel electrophoresis, which correspond to the degree of incorporation. These same conjugated molecules migrate faster then corresponding underivatized antibody on 4-12% native protein gel electrophoresis. The separation is more dramatic, but less well defined for higher order conjugates on native gels.

The extent of DNA conjugation was assessed by relative band densities for free and conjugated antibody, and varied from 50 to 100%, depending on the relative reactivity and exposure of lysine amine groups contained within the antibody structure. The degree of labeling was determined by the number and relative density of conjugate protein bands, and indicated a mixture of predominately single- and double- DNA labels per antibody.

Unreacted DNA was removed from the conjugate preparation by FPLC gel filtration chromatography employing a Superdex 200 HR 10/30 column (Amersham Pharmacia, Piscataway, NJ) equilibrated in phosphate-buffered saline. Unreacted antibody was removed by FPLC anion exchange chromatography using a Mono Q HR 5/5 column equilibrated in 20 MM Tris-Cl buffer, pH 8.0. and a 5%/min NaCl gradient to 1.0 M at 0.5 ml/min.

The conjugated DNA was rendered double-stranded by hybridization to a complementary 59-mer oligonucleotide:

5' -TGCTCGTATC GATCGCTGAT GCTAGCTAAT ATCGATCAGC AGCTAGTCAT

CGCTACGCA-3' (SEQ ID NO:18)

Excess complimentary DNA was removed by several passes through a Microcon YM- 100 centrifugal filter device.

### Example 3: Labeling Monoclonal Antibody With Biotin.

Capture MAb to PSA (A45120136P (Biospecific)) was labeled with biotin for immobilization on avidin-coated solid supports. Capture MAb (1 mg/ml) was reacted with 7.5 and 15-fold molar excess sulfo-NHS-LC-biotin (Pierce, Rockford, III.) in 0.1 M sodium phosphate, pH 8.0, for 2-4 hours at room temperature. The reaction yielded 3.6 and 7.3 moles biotin/mole MAb, respectively, as determined by reaction with HABA. The biotinylated capture MAb was purified on a desalting column, and tested for immune reactivity to prostate specific antigen (PSA) by ELISA.

### Example 4: Labeling 5' Amino DNA With Biotin.

Oligonucleotides synthesized to contain a 5' amino (C12) linkage were biotinylated by the addition of excess solid sulfo-NHS-LC-biotin to 1-5 mg/ml solution in 0.1 M sodium bicarbonate, pH 8.5. The reaction was allowed to proceed for 4 hours to overnight at room temperature. Biotinylated DNA was purified from the reaction mixture by HPLC using a C-18 reverse phase column eluted with a gradient of acetonitrile in water. Pure biotinylated oligonucleotides were dried by vacuum centrifugation and stored frozen.

### Example 5: Preparation Of Paramagnetic Micro Particles Coated With Cmab-Biotin.

Bio-Mag Streptavidin paramagnetic particles (Polysciences, Warrington, PA) or SeraMag-Streptavidin (Seradyn, Ramsey, MN) (5 mg in 500 µl) were placed in a 2 ml screw cap vial. Particles were collected to the side of the vial under a magnetic field, and the supernatant discarded. Particles were washed twice by magnetic collection and resuspension in 2 ml Phosphate Buffered Saline containing 0.05% Tween-20. Particles were resuspended in 1 ml blocking solution (PBS containing 1% bovine serum albumin, 0.1 mg/ml salmon sperm DNA and 0.1% sodium azide) and transferred to a 50 ml conical tube. To this, 24 ml of 10 nM biotinylated cMAb (labeled with biotin either directly, as in example 3, or via adouble-stranded DNA bridge) in blocking solution was added, followed by an incubation for one hour under gentle agitation at room temperature. Particles were washed three times by magnetic collection and resuspension in 25 ml PBS/Tween and once in PBS. The particles were resuspended in 2.5 ml blocking solution and stored at 4°C.

Similarly, Reacti-Bind NeutrAvidin Polystyrene microtiter plates (Pierce, Rockford, IL.) were also coated with cMAb via a biotin label. The degree of coating on the supports was monitored by loss of biotinylated MAb from solution as measured by bicinchoninic acid (Pierce BCA Protein Assay Reagent). Immobilized capture MAb was shown by protein gel electrophoresis to bind PSA and PSA reporter MAb-DNA complex.

### Example 6: Sandwich Immuno-PCR Assay For Prostate Specific Antigen.

Eighty (80) µl of 12.5 pM rMAb-DNA conjugate from Example 1 in blocking solution was added to each sample well in an iCycler plate (Bio-Rad, Hercules CA), followed by 20 µl of aserum sample or standard dilution series. The solution was gently mixed by repipetting. The plate was covered with sealer to prevent evaporation and incubated 2 hours at room temperature to form the first immune complex in solution

cMAb - biotin-coated magnetic microparticles (5 µl) were added and mixed by gentle repipetting. The plate was covered with sealer and incubated for 30 min under gentle agitation to capture the PSA immune complex onto particle.

Particles were collected to the sides of the wells under a magnetic field and the supernatant was drawn off using a pipette fitted with filter tips. The particles were resuspended in 120 µl TKMT (10 mM Tris, pH 8.0 containing 50 mM ACl,1.5 mM MgCl2, 0.05% Tween20) using filter tipped pipettes. This collection and resuspension of the particles was repeated an additional five times, increasing the volume of each successive wash by 20 µl. This ensured the removal of excess reporter MAb-DNA conjugate from the liquid meniscus on the walls of the well.

### Capture of the PSA Immune Complex Onto Particles.

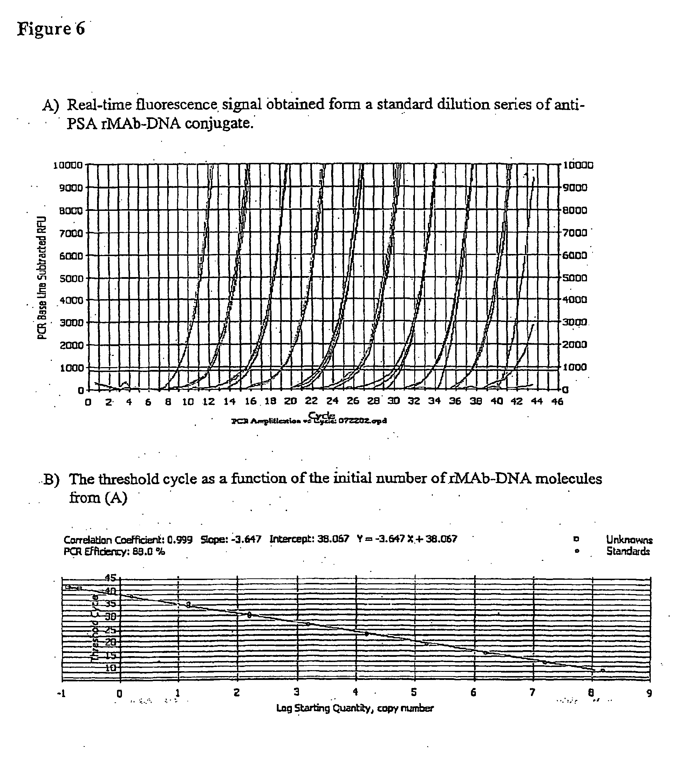

The particles were resuspended in 50 µl of PCR reaction (Platinum Quantitative PCR Super Mix, Invitrogen, Carlsbad, CA) containing SYBR green @ 1:30000 (Molecular Probes, Eugene, OR), 10 nM fluorescein, 0.2 µM each primer, and 0.5 x TKMT. The plate was covered with optical quality sealing tape and gently tapped to dislodge bubbles at the bottom of the wells. PCR amplification was carried out automatically for 40 cycles of 95°C melt for 15 sec and 62°C extension for 1 minute in an iCycler Instrument. The fluorescence intensity was monitored in real time and a cycle number was recorded for each sample that achieved a threshold level of signal. This threshold cycle number was then compared to values obtained for a standard dilution series of marker DNA. Assays of this format typically detected about 5000 molecules of PSA above background (signal in the absence of PSA). Figure 6 shows the results of a real-time PCR assay of marker DNA attached to rMAb for PSA.

### Example 7: Displacement Immuno-PCR Assay for Prostate Specific Antigens

Capturing antibody to PSA (Biospecific A45120136P) was conjugated to:
5'-NH3(C12)-TAGGACTTCAGACTGGGACGAT-3'(SEQ ID NO:2)
and hybridized to:
3'-ATCCTGAAGTCTGACCCTGCTAGCTCGAG-(C12)NH3-5'-biotin (SEQ ID NO:1)
then coated onto BioMag Streptavidin paramagnetic microparticles (lot 495594) at 50 pmol/mg. Prostate Specific Antigen (0 to 10⁶ molecules) was incubated for 2 hours at room temperature with 10 pM rMAb (Biospecific A215110136P) conjugated to: 
5'-NH3-(C12)-TGCGTAGCGA TGACTAGCTGC TGATCGATAT

TAGCTAGCAT CAGCGATCG ATACGAGCA-3' (SEQ ID NO:13)
 hybridized to 
3′-ACGCATCGCT ACTGATCGAC GACTAGCTAT AATCGATCGT AGTCGCTAGC
TATGCTCGT-5′ (SEQ ID NO:14)
 in 0.1 ml of blocking structure.

The hybridization structure is as indicated below: 
5'-NH3 (C12)-TGCGTAGCGA TGACTAGCTG CTGATCGATA
          |||||||||| |||||||||| ||||||||||
        3'-ACGCATCGCT ACTGATCGAC GACTAGCTAT

          TTAGCTAGCA TCAGCGATCG ATACGAGCA-3' (SEQ ID
        NO:13)
        .|||||||||| |||||||||| |||||||||
          AATCGATCGT AGTCGCTAGC TATGCTCGT-5' (SEQ ID
    NO:14)
 The immune complex was removed from solution within 30 min at room temperature by the addition of 5 µl suspension of coated microparticles (2 mg/ml). The microparticles were washed 6 times by repeated magnetic collection and resuspension in TKMT and divided into two sets.

One set was resuspended in 25 µl TKMT and subjected to real-time PCR analysis using the primer set:
5'-TGCGTAGCGA TGACTAGCTG CTG-3' (SEQ ID NO:15) and
5'-TGCTCGTATC GATCGCTGAT GCT-3' (SEQ ID NO:16)
The other set was resuspended in 25 µl of 1 µM displacer oligonucleotide of the sequence:
5'-GGACTTCAGA CTGGGACGAT CGAGCTC-3' (SEQ ID NO:3)
dissolved in TKMT. The particles were collected by magnetic field and the supernatant subjected to real-time PCR analysis using the same primers. The signal from each reaction was analyzed against a standard curve of rMAb-DNA conjugate.

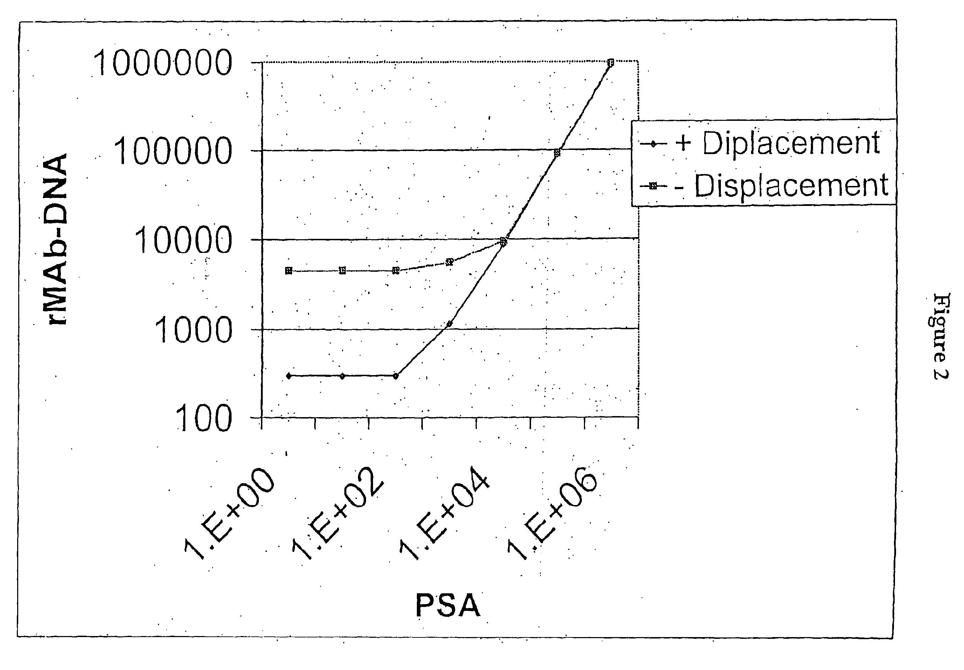

The results shown in Figure 2 indicate a background reduction of approximately 10-fold by the displacement of the immune complex from the paramagnetic microparticles, resulting in a commensurate improvement in ultimate assay sensitivity.

### Example 8: Displacement Reactions.

**Endonuclease Cleavage:** A dissociable linker connecting capture MAb to biotin was constructed using a 26 base pair double-stranded DNA containing restriction endonuclease sites (SEQ ID NO:17). The 26- base oligonucleotides, each containing a 5' amino linkage were synthesized to contain both Hind III and Eco RI restriction sites when hybridized. The amino function on one DNA strand was derivatized with sulfo-NHS-LC-biotin. The second strand was conjugated to capture MAb against PSA followed by hybridization to the biotinylated complementary strand. The biotinylated capture MAb conjugate was fully bound by immobilized avidin (or streptavidin) and released by digestion with a restriction endonuclease. Both Hind III and Eco RI were employed, with Eco RI digestion being more efficient. A captured PSA-reporter MAb immune complex was effectively cleaved from the paramagnetic support by Eco RI treatment at 37 °C for 15 min. These conditions, however, eluted nonspecific reporter MAb-DNA conjugate as well. Sensitivity of a sandwich Immuno-PCR assay (Example 6) improved marginally for the detection of PSA when the immune complex was displaced form the solid support by restriction cleavage of the DNA linker. Similar results were obtained with streptavidin- or avidin-coated microtiter plates.

5' CAGGTAGAAT TCCTAAGCTT CAGGCT (SEQ ID NO:17)
3' GTCCATCTTA AGGATTCGAA GTCCGA
Eco R1 Hind III

**Displacement**: Another means to reduce background during the final PCR reaction employed a dissociable double-strand DNA linker between the capture MAb and the biotin used to link to the solid support. Peptide nucleic acid (PNA) is known to form extremely tight hybrids with complimentary DNA and was used to displace a hybridized DNA strand of the same sequence. The capture MAb was biotinylated via a linker arm composed of double-stranded DNA, which would dissociate in the presence of excess complimentary PNA.

**Protein Nucleic Acid**: Peptide nucleic acid (PNA) is known to form extremely tight hybrids with complimentary DNA and was used to displace a hybridized DNA strand of the same sequence.

A 22 base DNA anchor sequence was synthesized with a 5' amino function and biotinylated. A 15 base DNA capture sequence, complimentary to the 3' end of the anchor sequence, was synthesized with a 5' amino function and conjugated to the capture MAb. Capture MAb conjugate was hybridized to biotinylated anchor sequence, leaving a 7 base single-strand region at the 5' end of the anchor. Identical 18 base displacer sequences of PNA and DNA were synthesized to be complimentary to the 5' end of the anchor sequence. Hybridization of the displacer sequence to the 7 base open region on the anchor probe would "displace" the hybridized capture sequence, releasing the immune complex, and thus template DNA, into solution. The biotinylated anchor sequence and hybridized displacer sequence, as well as surface bound reporter MAb-DNA, would remain with the avidin-coated support. The supernatant would be transferred to a new vessel for PCR amplification. This system is called capture/displacement (*see, e.g.,* Figure 1).

The capture sequence was also conjugated to alkaline phosphatase in order monitor and optimize the capture/displacement system by following enzyme activity. This arrangement of DNA sequences demonstrated the capture and release from avidin particles and microtiter wells. Surprisingly, it was found that the DNA displacer sequence outperformed the PNA sequence in isotonic saline buffer, while the opposite was true for low salt conditions. Addition of 1 µM displacer DNA to alkaline phosphatase immobilized through double-stranded biotinylated DNA was released completely into solution within seconds. The system was equally efficient when applied to the immuno-PCR assay. Displacement conditions using DNA were identical to prior wash conditions and compatible with PCR. Capture/displacement resulted in a clear reduction in background without affecting signal.

**Kinetics of Displacement:** Alkaline Phosphatase was conjugated to a 15 base capture oligonucleotide (GTGCTTCCTC TTTCT (SEQ ID NO:8)) via a 5' amino (C12) linker arm using disuccidimidyl suberate. The DNA was hybridized to a complimentary 22 base anchor oligonucleotide (5'-GACACACAGA AAGAGGAAGC AC (SEQ ID NO:9)) labeled with biotin through a 5' amino linker arm, resulting in alkaline phosphatase labeled with biotin through a partially double-stranded DNA linker. The alkaline phosphatase/DNA conjugate was dissolved in PBS containing 1 MM MgC12, 0.1 mM ZnC12, and 0.1% BSA. Fifty micrograms avidinylated paramagnetic microparticles (BioMag Streptavidin, Polysciences, Inc., Warrington PA, 18976) were washed with the same buffer and combined with 2.5 µg alkaline phosphatase/DNA conjugate in 0.5 ml buffer. Approximately half of the enzyme activity was found bound to the microparticles as determined by monitoring the change in optical density at 405 nm in the presence of 1 mg/ml para-nitrophenyl phosphate dissolved in 2.4 M diethanolamine, 57 µM MgC12, pH 10. The particles were washed 4 times in buffer and divided into three equal amounts. The release of enzyme activity from the microparticles into solution was monitored as a function of time following the addition of displacement oligonucleotide or protein nucleic acid (PNA) (5'-CTTCCTCTTTCTGTGTGT (DNA backbone: SEQ ID NO:6 and PNA backbone: SEQ ID NO: 11)).

**Table 1.**

| | Time (min) | Soluble Enzyme Activity |
|---|---|---|
| Control (no addition) | 5 | 1.3% |
| | 10 | 1.4% |
| | 20 | 1.4% |
| | 30 | 1.5% |
| PNA addition | 1 | 50% |
| | 3 | 65% |
| | 5 | 80% |
| | 10 | 90% |
| | 20 | 94% |
| | 30 | 97% |
| DNA addition | 1 | 82% |
| | 3 | 91% |
| | 5 | 97% |
| | 10 | 98% |
| | 20 | 98% |
| | 30 | 98% |

This observation was repeated several times using Alkaline Phosphatase conjugated to (5'-GCTACTTGAC TTCGAGTGCT TCCTCTTTCT (SEQ ID No:5)) /(5'-biotin-GACACACAGA AAGAGGAAGC AC (SEQ ID NO:9)) and removed with the same displacer at 1 µM. In all cases, almost 100% of the bound enzyme activity was displaced back into solution within 5 minutes.

Displacement kinetics can also be observed with antibody conjugates to PSA displaced back into solution. The presence of low amounts of antibody in solution is determined by ELISA. The antibody against PSA is captured onto PSA-coated plates, then coated with a secondary HRP-Ab. The HRP activity is proportional to the presence of the first antibody.

Microtiter plates coated with avidin (Reacti-Bind Neutravidin Coated Polystyrene Strip Plate with SuperBlock Blocking Buffer, Pierce, Rockford, IL 61105) were incubated for 30 minutes with 10 nM anti-PSA Ab (unlabeled, labeled with biotin directly, or conjugated to the capture/biotinylated anchor oligonucleotide hybrid). The wells were washed three times in PBS containing 0.05% Tween. The antibody conjugate was displaced by the addition of 1 µM displacer oligonucleotide and the corresponding PNA in 10 mM Tris, pH 8, containing 0 to 500 mM salt. The wells were drained and washed as a function of incubation time. Anti-mouse antibody labeled with HRP was added to the wells and incubated for 1.5 hours. Anti-PSA antibody was determined by HRP activity measured at 490 nm in the presence of OPD/peroxide in a Molecular Devices Corp. plate reader. Note that although PNA is used in this example, DNA and RNA molecules may also be used as a displacer nucleic acid.

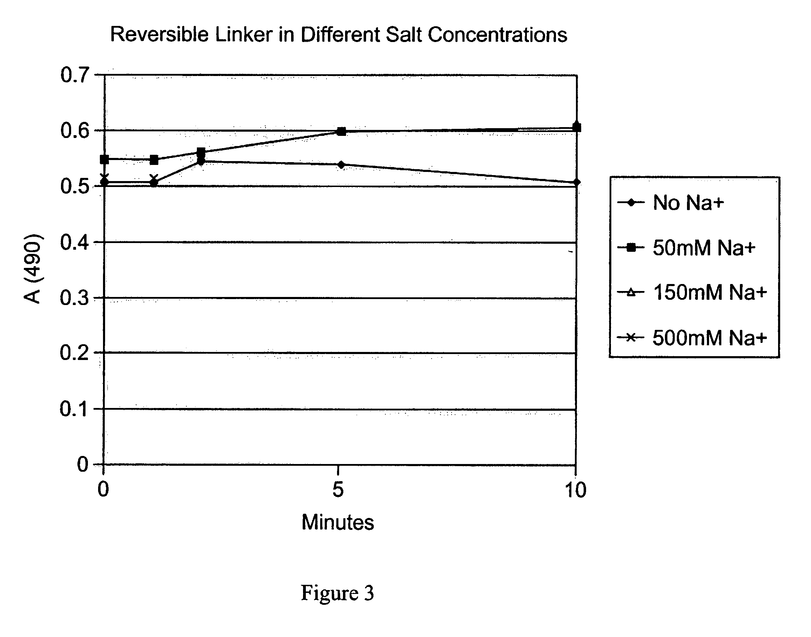

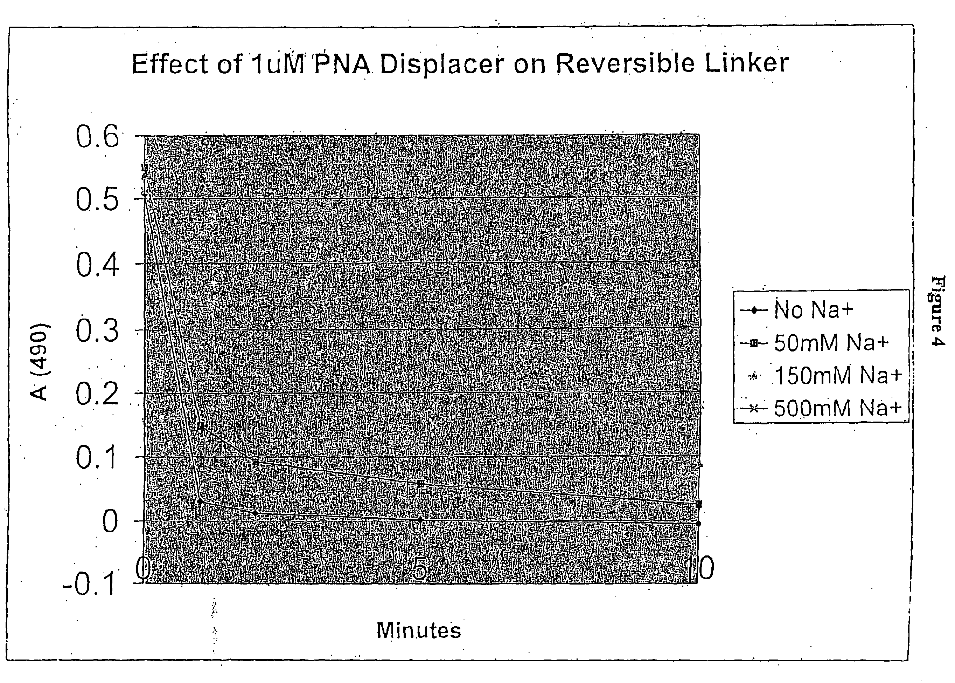

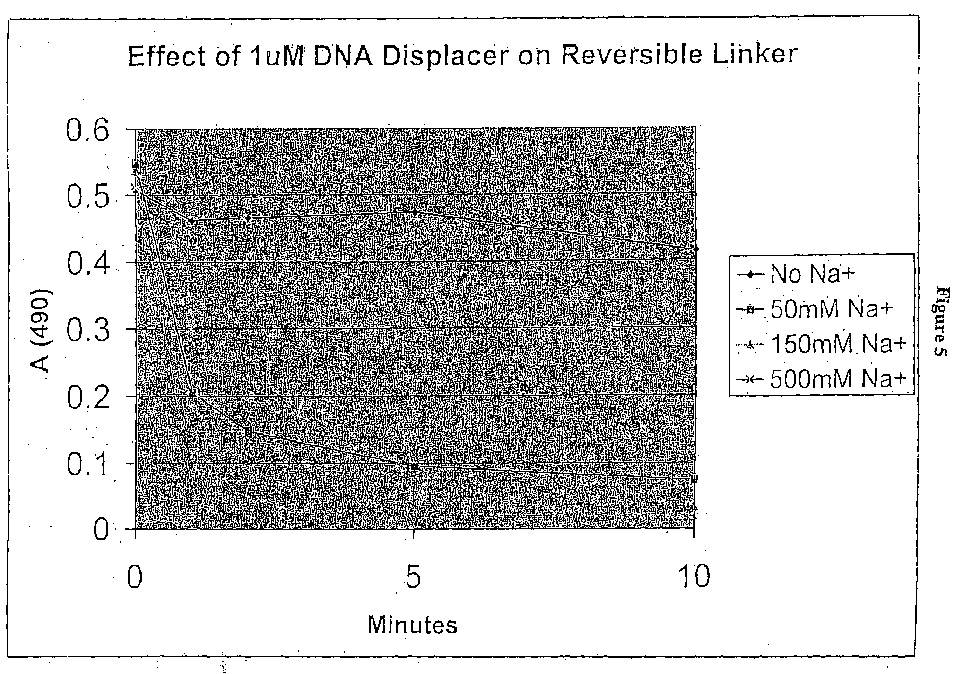

Figure 3 shows that the capture/anchor hybrid is stable under the salt conditions of the buffer. The antibody/biotinylated DNA conjugate remains bound to the well, as well as controls. Figure 4 shows the effect of PNA displacement on the bound antibody/biotinylated DNA conjugate. At low ionic strength, PNA displacement is very effective, removing the antibody within a few minutes. It is not as effective at high salt. Figure 5 shows displacement with a molecule of DNA. It is very effective at high salt, and ineffective in the absence of salt.

**Displacement/Recapture:** If necessary, the capture/displacement concept could be extended to include another round of capture and release. To test this hypothesis, longer, 30 base capture sequence was synthesized, containing a second region complimentary to a second capture sequence. Immune complex displaced into solution from the first avidin coated support would be recaptured by solution hybridization to a second biotinylated sequence, followed by exposure to a fresh avidin solid support. Each capture/release step improves sensitivity by removing nonspecific reporter MAb-DNA. The capture DNA sequence for the capture/displacement/recapture system was conjugated to alkaline phosphatase and capture MAb. The system was analyzed for efficacy by following enzyme activity captured, displaced and recaptured onto paramagnetic particles. The MAb conjugate was then incorporated into the immuno-PCR assay. In preferred embodiments of the present invention, only one round of capture/displacement is being explored. For the instant invention, capture/displacement is used. However, if required, a additional capture /displacement steps may be used in the instant invention.

**Table 2: Sequences for Capture/Displacement NADIA**

| |
|---|
| Anchor sequence for Biotinylation: (29mer) (SEQ ID NO: 1) |
| 5' NH2- (C₁₂) -GAGCTCGATC GTCCCAGTCT GAAGTCCTA 3' |
| 9470 g/mole; e = 2.75E5; 1 od = 34.4 µg/ml or 3.64. µM |
| Capture sequence for antibody labeling (22 mer) (SEQ ID NO:2) |
| 5'NH2 - (C₁₂) TAGGACTTCA GACTGGGACG AT 3' |
| 7261 g/mole; e = 2.18E5; 1 od = 33.2 µg/m or 4.58 µM |
| Displacer sequence (27 mer) (SEQ ID NO:3) |
| 5' GGACTTCAGA CTGGGACGAT CGAGCTC 3' |
| 8897 g/mole; e = 2.60E5; 1 od = 34.2 µg/ml or 3.85 µM |
| 1 st capture (SEQ ID NO:4) |
| 3' CACGAAGGAG AAAGACACAC AG 5' - (C12) -NH3-biotin |
| MW = 9574, e = 2.59E5, 1OD = 37 µg/ml or 3.86 µM |
| 30mer (SEQ ID NO:5) |
| Capture Ab-5' NH3-(C12)-GCTACTTGAC TTCGAGTGCT TCCTCTTTCT |
| Removal PNA (SEQ ID NO: 11) |
| 5' CTTCCTCTTT CTGTGTGT 3' |
| 2nd capture |
| 3' CGATGAACTG AAGCTCAC- (C12) -NH2 5'-biotin or 5' NH₃- (C₁₂) -CACTCGAA GTCAAGTAGC (SEQ ID NO:7) |
| MW = 5782, e = 1.785E5, 1OD = 32.4 µg/ml or 5.6 µM |

## Claims

1. A method for detecting a non-nucleic acid analyte present in a sample, the method comprising the steps of:
(i) contacting a sample comprising a non-nucleic acid analyte with a reporter conjugate, wherein the reporter conjugate comprises:
(a) a first receptor capable of specifically binding the analyte; and
(b) a nucleic acid marker;
(ii) allowing the binding of the analyte to the reporter conjugate, thereby forming an analyte-dependent reporter complex;
(iii) contacting the analyte-dependent reporter complex with a second receptor for the analyte, wherein the second receptor is attached to a solid support via a nucleic acid bridge of predetermined sequence consisting of two at least partially complementary nucleic acid strands one of which is attached to the solid support;
(iv) allowing the binding of the second receptor to the analyte-dependent reporter complex, thereby forming an analyte-dependent reporter/second receptor complex attached to the solid support;
(v) eluting the analyte-dependent reporter/second receptor complex from the solid support with a displacer nucleic acid having a sequence at least partially complementary to one of the nucleic acid bridge strands; and
(vi) specifically detecting the presence of the nucleic acid marker in the eluted analyte-dependent reporter/second receptor complex, wherein the detection of nucleic acid marker indicates the presence of the analyte in the sample.

2. The method of claim 1, wherein detecting the presence of the nucleic acid marker comprises:
(i) contacting the eluted analyte-dependent reporter/second receptor complex with a nucleic acid replication composition capable of replicating the nucleic acid marker sequence,
(ii) replicating the nucleic acid marker; and
(iii) visualizing the replicated nucleic acid marker by ethidium bromide staining.

3. The method of claim 2, wherein the nucleic acid marker is replicated with a polymerase.

4. The method of claim 3, wherein the polymerase is selected from the group consisting of a DNA polymerase, a RNA polymerase, a transcriptase, and a Q-beta polymerase.

5. The method of claim 2, wherein the replication composition comprises reagents and primers for conducting a polymerase chain reaction, and wherein replicating the nucleic acid marker comprises amplifying the nucleic acid marker by the polymerase chain reaction.

6. The method of claim 1, wherein the first and second receptors are antibodies and the non-nucleic acid analyte is an antigen or a hapten.

7. The method of claim 5, wherein the polymerase chain reaction is a real-time polymerase chain reaction.

8. The method of claim 1, wherein the analyte is PSA.

9. The method of claim 1, wherein the sample is a serum sample and the analyte is present at a concentration of less than 10 picograms per milliliter.

10. The method of claim 1, further comprising the step of washing the analyte-dependent reporter/second receptor complex attached to the solid support formed in step (iv) prior to eluting the analyte-dependent reporter/second receptor complex from the solid support with a displacer nucleic acid in step (v).

11. The method of claim 1, wherein the steps comprise:
(i) contacting a sample comprising a multiplicity of different non-nucleic acid analytes with a multiplicity of reporter conjugates, wherein each reporter conjugate comprises: (a) a first receptor capable of specifically binding one of the multiplicity of non-nucleic acid analytes in the sample; and (b) a nucleic acid marker, wherein each reporter conjugate binds specifically to a different analyte and comprises a nucleic acid that is distinguishable from the other nucleic acids in the multiplicity of reporter conjugates;
(ii) allowing the binding of the multiplicity of non-nucleic acid analytes in the sample to the multiplicity of receptor conjugates, thereby forming a multiplicity of analyte-dependent reporter complexes;
(iii) contacting the multiplicity of analyte-dependent reporter complexes with a multiplicity of second receptors, wherein the multiplicity of second receptors is collectively capable of binding to the multiplicity of analytes in the sample, and wherein the second receptors are attached to a solid support via a nucleic acid bridge of predetermined sequence;
(iv) allowing the binding of the second receptors to the analyte-dependent reporter complexes, thereby forming a multiplicity of analyte-dependent reporter/second receptor complexes attached to the solid support;
(v) eluting the multiplicity of analyte-dependent reporter/second receptor complexes from the solid support with at least one displacer nucleic acid; and
(vi) specifically detecting the presence of the nucleic acid markers in the eluted multiplicity of analyte-dependent reporter/second receptor complexes by
(a) contacting the eluted multiplicity of analyte- dependent reporter/second receptor complexes with a nucleic acid replication composition capable of replicating the nucleic acid markers;
(b) simultaneously replicating all of the distinguishable nucleic acid markers; and
(c) visualizing the replicated nucleic acid marker by ethidium bromide staining, wherein the detection of each distinguishable nucleic acid marker indicates the presence of the corresponding analyte in the sample.

12. The method of claim 11, wherein the nucleic acid markers are replicated with a polymerase.

13. The method of claim 12 wherein the polymerase is selected from the group consisting of a DNA polymerase, a RNA polymerase, a transcriptase, and a Q-beta polymerase.

14. The method of claim 11, wherein the nucleic acid replication composition comprises reagents and primers for conducting a polymerase chain reaction, and wherein replicating the nucleic acid markers comprises amplifying the nucleic acid markers in a polymerase chain reaction.

15. The method of claim 11, wherein the multiplicity of first and second receptors are antibodies and the multiplicity of non-nucleic acid analytes comprise antigens, haptens or a combination thereof

16. The method of claim 14, wherein the polymerase chain reaction is a real-time polymerase chain reaction.

17. The method of claim 11, wherein one of the multiplicity of non-nucleic acid analytes is PSA.

18. The method of claim 11, wherein the sample is a serum sample and at least one of the multiplicity of non-nucleic acid analytes is present at a concentration of less than 10 pictograms per milliliter.

19. The method of claim 1 further comprising:
In step (ii) providing an antibody/analyte complex, wherein the 5'end of a first nucleic acid is conjugated to the antibody, the analyte comprises an antigen recognized by the antibody, and the analyte is specifically bound to the antibody through the antigen combining site of the antibody;
In step (iii) providing a solid support comprising a second nucleic acid, wherein the 5'end of the second nucleic acid is bound to the solid support, wherein the second nucleic acid is substantially complementary to the first nucleic acid over its 3'terminus;
In step (iv) binding the antibody/analyte complex to the solid support by means of a double-stranded nucleic acid bridge, wherein the nucleic acid bridge is formed by hybridization of the unbound ends of the first and second nucleic acids; and
In step (v) eluting the antibody/analyte complex from the solid support by means of a displacer nucleic acid.

20. The method of claim 1, wherein step (v) comprises the step of eluting the non-nucleic acid analyte-dependent reporter/second receptor complex that is attached to a solid support by means of a nucleic acid bridge by contacting the analyte-dependent reporter/second receptor complex with the displacer nucleic acid under conditions that favor displacement of the analyte-dependent reporter/second receptor complex with the displacer nucleic acid.

## Patentansprüche

1. Verfahren zum Nachweis eines nicht-Nukleinsäure-Analyts in einer Probe, das Verfahren umfasst die Schritte:
(i) Kontaktieren einer Probe, die einen nicht-Nukleinsäure-Analyt umfasst, mit einem Reporter-Konjugat, wobei das Reporter-Konjugat umfasst:
(a) einen ersten Rezeptor, der in der Lage ist spezifisch an den Analyt zu binden; und
(b) einen Nukleinsäuremarker;
(ii) Erlauben, dass der Analyt an das Reporter-Konjugat bindet, wodurch ein Analyt-abhängiger Reporter-Komplex gebildet wird;
(iii) in Kontakt bringen des Analyt-abhängigen Reporter-Komplexes mit einem zweiten Rezeptor, der den Analyt bindet, wobei der zweite Rezeptor über eine Nukleinsäurebrücke einer vorher festgelegten Sequenz, die aus zwei mindestens teilweise komplementären Nukleinsäuresträngen bestehen, wobei einer dieser Stränge auf dem festen Träger angebracht ist, auf einem festen Träger angebracht ist;
(iv) Erlauben, dass der zweite Rezeptor an den Analyt-abhängigen Reporter-Komplex bindet, wodurch ein Analyt-abhängiger Reporter/zweiter Rezeptor-Komplex, der an den festen Träger gebunden ist, gebildet wird;
(v) Eluieren des Analyt-abhängigen Reporter/zweiter Rezeptor-Komplexes vom festen Träger durch eine Verdrängungs-Nukleinsäure, die eine Sequenz besitzt, die mindestens teilweise zu einem der Stränge aus der Nukleinsäurebrücke komplementär ist; und
(vi) spezifisches Nachweisen der Anwesenheit des Nukleinsäuremarkers in dem eluierten Analyt-abhängigen Reporter/zweiter Rezeptor-Komplex, wobei der Nachweis des Nukleinsäuremarkers die Anwesenheit des Analyts in der Probe anzeigt.

2. Das Verfahren nach Anspruch 1, wobei das Nachweisen der Anwesenheit des Nukleinsäuremarkers umfasst:
(i) Kontaktieren des eluierten Analyt-abhängigen Reporter/zweiter Rezeptor-Komplexes mit einer Nukleinsäure-Replikationszusammensetzung, die in der Lage ist die Nukleinsäuremarker-Sequenz zu replizieren;
(ii) Replizieren des Nukleinsäuremarkers; und
(iii) Visualisieren des replizierten Nukleinsäuremarkers durch Ethidiumbromid-Färbung.

3. Das Verfahren nach Anspruch 2, wobei der Nukleinsäuremarker mit einer Polymerase repliziert wird.

4. Das Verfahren nach Anspruch 3, wobei die Polymerase aus der Gruppe bestehend aus einer DNA-Polymerase, einer RNA-Polymerase, einer Transkriptase und einer Q-beta Polymerase ausgewählt ist.

5. Das Verfahren nach Anspruch 2, wobei die Replikationszusammensetzung Reagenzien und Primer um eine Polymerase-Ketten-Reaktion durchzuführen, umfasst und wobei das Replizieren des Nukleinsäuremarkers das Amplifizieren des Nukleinsäuremarkers mittels der Polymerase-Ketten-Reaktion umfasst.

6. Das Verfahren nach Anspruch 1, wobei der erste und der zweite Rezeptor Antikörper sind und der nicht-Nukleinsäure-Analyt ein Antigen oder ein Hapten ist.

7. Das Verfahren nach Anspruch 5, wobei die Polymerase-Ketten-Reaktion eine Echtzeit-Polymerase-Ketten-Reaktion ist.

8. Das Verfahren nach Anspruch 1, wobei der Analyt PSA ist.

9. Das Verfahren nach Anspruch 1, wobei die Probe eine Serumprobe ist und der Analyt in einer Konzentration von weniger als 10 Pikogramm pro Milliliter anwesend ist.

10. Das Verfahren nach Anspruch 1, ferner umfassend einen Waschschritt des Analyt-abhängigen Reporter/zweiter Rezeptor-Komplexes, der an den festen Träger gekoppelt ist und in Schritt (iv) ausgebildet wird, bevor der Analyt-abhängige Reporter/zweiter Rezeptor-Komplex vom festen Träger durch eine Verdrängungs-Nukleinsäure in Schritt (v) eluiert wird.

11. Das Verfahren nach Anspruch 1, umfassend die Schritte:
(i) Kontaktieren einer Probe, die eine Vielzahl von verschiedenen nicht-Nukleinsäure-Analyten umfasst, mit einer Vielzahl von Reporter-Konjugaten, wobei jedes Reporter-Konjugat umfasst: (a) einen ersten Rezeptor, der in der Lage ist spezifisch an einen der vielzähligen Analyten der Probe zu binden; und (b) einen Nukleinsäuremarker, wobei jedes Reporter-Konjugat spezifisch an einen anderen Analyt bindet und eine Nukleinsäure umfasst, die sich von den anderen Nukleinsäuren innerhalb der Vielzahl der Reporter-Konjugate unterscheidet;
(ii) Erlauben, dass die Vielzahl der nicht-Nukleinsäure-Analyten in der Probe an die Vielzahl der Reporter-Konjugate bindet, wodurch eine Vielzahl von Analyt-abhängige Reporter-Komplexen ausgebildet wird;
(iii) in Kontakt bringen einer Vielzahl von Analyt-abhängigen Reporter-Komplexen mit einer Vielzahl von zweiten Rezeptoren, wobei die Vielzahl der zweiten Rezeptoren insgesamt in der Lage ist, an die Vielzahl der Analyten in der Probe zu binden, und wobei der zweite Rezeptor über eine Nukleinsäurebrücke einer vorher festgelegten Sequenz auf einem festen Träger angebracht ist;
(iv) Erlauben, dass die zweiten Rezeptoren an die Analyt-abhängigen Reporter-Komplexe binden, wodurch eine Vielzahl von Analyt-abhängigen Reporter/zweiter Rezeptor-Komplexen, die an den Träger gebunden sind, gebildet werden;
(v) Eluieren der Vielzahl der Analyt-abhängigen Reporter/zweiter Rezeptor-Komplexe vom Träger durch mindestens eine Verdrängungs-Nukleinsäure; und
(vi) spezifisches Nachweisen der Anwesenheit der Nukleinsäuremarker in der Vielzahl der eluierten Analyt-abhängigen Reporter/zweiter Rezeptor-Komplexe durch
(a) in Kontakt bringen der eluierten vielzähligen Analyt-abhängigen Reporter/zweiter Rezeptor-Komplexe mit einer Nukleinsäure-Replikationszusammensetzung, die in der Lage ist die Nukleinsäuremarker-Sequenz zu replizieren;
(b) gleichzeitiges Replizieren aller verschiedenen Nukleinsäuremarker; und
(c) Darstellen der replizierten Nukleinsäuremarker durch Ethidiumbromid-Färbung, wobei der Nachweis jedes einzelnen unterschiedlichen Nukleinsäuremarkers die Anwesenheit des entsprechenden Analyts in der Probe anzeigt.

12. Das Verfahren nach Anspruch 11, wobei die Nukleinsäuremarker mit einer Polymerase repliziert werden.

13. Das Verfahren nach Anspruch 12, wobei die Polymerase aus der Gruppe bestehend aus einer DNA-Polymerase, einer RNA-Polymerase, einer Transkriptase und einer Q-beta Polymerase ausgewählt ist.

14. Das Verfahren nach Anspruch 11, wobei die Nukleinsäure-Replikationszusammensetzung Reagenzien und Primer um eine Polymerase-Ketten-Reaktion durchzuführen umfasst und wobei das Replizieren der Nukleinsäuremarker das Amplifizieren der Nukleinsäuremarker mittels der Polymerase-Ketten-Reaktion umfasst.

15. Das Verfahren nach Anspruch 11, wobei die Vielzahl der ersten und zweiten Rezeptoren Antikörper sind und die Vielzahl der nicht-Nukleinsäure-Analyten Antigene, Haptene oder eine Kombination davon umfasst.

16. Das Verfahren nach Anspruch 14, wobei die Polymerase-Ketten-Reaktion eine Echtzeit-Polymerase-Ketten-Reaktion ist.

17. Das Verfahren nach Anspruch 11, wobei einer der vielzähligen nicht-Nukleinsäure-Analyten PSA ist.

18. Das Verfahren nach Anspruch 11, wobei die Probe eine Serumprobe ist und mindestens einer der nicht-Nukleinsäure-Analyten in einer Konzentration von weniger als 10 Pikogramm pro Milliliter anwesend ist.

19. Das Verfahren nach Anspruch 1 ferner umfassend:
In Schritt (ii): Bereitstellen eines Antikörper/Analyt-Komplexes, wobei das 5'-Ende einer ersten Nukleinsäure an den Antikörper gebunden ist, der Analyt ein Antigen, das durch den Antikörper erkannt wird, umfasst, und der Analyt über die Antigen bindende Stelle des Antikörpers spezifisch an den Antikörper gebunden wird;
In Schritt (iii): Bereitstellen eines festen Trägers, der eine zweite Nukleinsäure umfasst, wobei das 5'-Ende der zweiten Nukleinsäure an den festen Träger gebunden ist, wobei die zweite Nukleinsäure über ihren 3'-Terminus im Wesentlichen komplementär zu der ersten Nukleinsäure ist; und
In Schritt (iv): Binden des Antikörper/Analyt-Komplexes an den festen Träger durch eine doppelsträngige Nukleinsäurebrücke, wobei die Nukleinsäurebrücke durch die Hybridisierung der ungebundenen Enden der ersten und zweiten Nukleinsäure ausgebildet wird; und
In Schritt (v): Eluieren des Antikörper/Analyt-Komplexes vom festen Träger durch eine Verdrängungs-Nukleinsäure.

20. Das Verfahren nach Anspruch 1, wobei Schritt (v) den Schritt des Eluierens des Analyt-abhängigen Reporter/zweiter Rezeptor-Komplexes, der an den Träger durch eine Nukleinsäurebrücke gebunden ist, durch in Kontakt bringen des Analyt-abhängigen Reporter/zweiter Rezeptor-Komplexes mit einer Verdrängungs-Nukleinsäure unter Bedingungen, die eine Verdrängung des Analyt-abhängigen Reporter/zweiter Rezeptor-Komplexes mit der Verdrängungs-Nukleinsäure begünstigen, umfasst.

## Revendications

1. Procédé de détection d'un analyte d'acide non nucléique présent dans un échantillon, le procédé comprenant les étapes suivantes :
(i) la mise en contact d'un échantillon comprenant un analyte non d'acide nucléique avec un conjugué rapporteur, dans lequel le conjugué rapporteur comprend :
(a) un premier récepteur capable de lier spécifiquement l'analyte ; et
(b) un marqueur d'acide nucléique ;
(ii) la possibilité de la liaison de l'analyte au conjugué rapporteur, formant de cette manière un complexe de rapporteur dépendant de l'analyte ;
(iii) la mise en contact du complexe de rapporteur dépendant de l'analyte avec un second récepteur pour l'analyte, dans lequel le second récepteur est fixé à un support solide par l'intermédiaire d'un pont d'acide nucléique d'une séquence prédéterminée constitué de deux brins d'acide nucléique au moins partiellement complémentaires dont l'un est fixé au support solide ;
(iv) la possibilité de la liaison du second récepteur au complexe de rapporteur dépendant de l'analyte, formant de cette manière un complexe de rapporteur dépendant de l'analyte/second récepteur fixé au support solide ;
(v) l'élution du complexe de rapporteur dépendant de l'analyte/second récepteur à partir du support solide avec un acide nucléique de déplacement ayant une séquence au moins partiellement complémentaire de l'un des brins du pont d'acide nucléique ; et
(vi) la détection spécifique de la présence du marqueur d'acide nucléique dans le complexe de rapporteur dépendant de l'analyte/second récepteur élué, dans lequel la détection du marqueur d'acide nucléique indique la présence de l'analyte dans l'échantillon.

2. Procédé selon la revendication 1, dans lequel la détection de la présence du marqueur d'acide nucléique comprend :
(i) la mise en contact du complexe de rapporteur dépendant de l'analyte/ second récepteur élué avec une composition de réplication d'acide nucléique capable de répliquer la séquence du marqueur d'acide nucléique,
(ii) la réplication du marqueur d'acide nucléique ; et
(iii) la visualisation du marqueur d'acide nucléique répliqué par une coloration au bromure d'éthidium.

3. Procédé selon la revendication 2, dans lequel le marqueur d'acide nucléique est répliqué avec une polymérase.

4. Procédé selon la revendication 3, dans lequel la polymérase est choisie dans le groupe constitué par une ADN polymérase, une ARN polymérase, une transcriptase et une polymérase Q-bêta.

5. Procédé selon la revendication 2, dans lequel la composition de réplication comprend des réactifs et des amorces permettant de conduire une réaction en chaîne de la polymérase, et dans lequel la réplication du marqueur d'acide nucléique comprend l'amplification du marqueur d'acide nucléique par la réaction en chaîne de la polymérase.

6. Procédé selon la revendication 1, dans lequel les premier et second récepteurs sont des anticorps et que l'analyte d'acide non nucléique est un antigène ou un haptène.

7. Procédé selon la revendication 5, dans lequel la réaction en chaîne de la polymérase est une réaction en chaîne de la polymérase en temps réel.

8. Procédé selon la revendication 1, dans lequel l'analyte est le PSA.

9. Procédé selon la revendication 1, dans lequel l'échantillon est un échantillon de sérum et l'analyte est présent à une concentration inférieure à 10 picogrammes par millilitre.

10. Procédé selon la revendication 1, comprenant en outre l'étape de lavage du complexe de rapporteur dépendant de l'analyte/second récepteur fixé au support solide formé dans l'étape (iv) avant l'élution du complexe de rapporteur dépendant de l'analyte/second récepteur à partir du support solide avec un acide nucléique de déplacement dans l'étape (v).

11. Procédé selon la revendication 1, dans lequel les étapes comprennent :
(i) la mise en contact d'un échantillon comprenant une multiplicité de différents analytes d'acides non nucléiques avec une multiplicité de conjugués rapporteurs, dans lequel chaque conjugué rapporteur comprend :
(a) un premier récepteur capable de lier spécifiquement l'un de la multiplicité d'analytes d'acides non nucléiques dans l'échantillon ; et (b) un marqueur d'acide nucléique, dans lequel chaque conjugué rapporteur se lie spécifiquement à un analyte différent et comprend un acide nucléique qui peut être distingué des autres acides nucléiques dans la multiplicité de conjugués rapporteurs ;
(ii) la possibilité de la liaison de la multiplicité d'analytes d'acides non nucléiques dans l'échantillon à la multiplicité de conjugués rapporteurs, formant de cette manière une multiplicité de complexes de rapporteurs dépendants de l'analyte ;
(iii) la mise en contact de la multiplicité de complexes de rapporteurs dépendants de l'analyte avec une multiplicité de seconds récepteurs, dans lequel la multiplicité de seconds récepteurs est collectivement capable de se lier à la multiplicité d'analytes dans l'échantillon, et dans lequel les seconds récepteurs sont fixés à un support solide par l'intermédiaire d'un pont d'acide nucléique d'une séquence prédéterminée ;
(iv) la possibilité de la liaison des seconds récepteurs aux complexes de rapporteurs dépendants de l'analyte, formant de cette manière une multiplicité de complexes de récepteurs dépendants de l'analyte/seconds récepteurs fixés au support solide ;
(v) l'élution de la multiplicité de complexes de rapporteurs dépendants de l'analyte/seconds récepteurs à partir du support solide avec au moins un acide nucléique de déplacement ; et
(vi) la détection spécifique de la présence des marqueurs d'acides nucléiques dans la multiplicité de complexes de rapporteurs dépendants de l'analyte/seconds récepteurs élués par
(a) la mise en contact de la multiplicité de complexes de rapporteurs dépendants de l'analyte/seconds récepteurs élués avec une composition de réplication d'acide nucléique capable de répliquer les marqueurs d'acides nucléiques ;
(b) la réplication simultanée de tous les marqueurs d'acides nucléiques pouvant être distingués ; et
(c) la visualisation des marqueurs d'acides nucléiques répliqués par une coloration au bromure d'éthidium, dans lequel la détection de chaque marqueur d'acide nucléique pouvant être distingué indique la présence de l'analyte correspondant dans l'échantillon.

12. Procédé selon la revendication 11, dans lequel les marqueurs d'acides nucléiques sont répliqués avec une polymérase.

13. Procédé selon la revendication 12, dans lequel la polymérase est choisie dans le groupe constitué par une ADN polymérase, une ARN polymérase, une transcriptase et une polymérase Q-bêta.

14. Procédé selon la revendication 11, dans lequel la composition de réplication d'acide nucléique comprend des réactifs et des amorces permettant de conduire une réaction en chaîne de la polymérase, et dans lequel la réplication des marqueurs d'acides nucléiques comprend l'amplification des marqueurs d'acides nucléiques dans une réaction en chaîne de la polymérase.

15. Procédé selon la revendication 11, dans lequel la multiplicité des premier et second récepteurs sont des anticorps et la multiplicité des analytes d'acides non nucléiques comprennent des antigènes, des haptènes ou une combinaison de ceux-ci.

16. Procédé selon la revendication 14, dans lequel la réaction en chaîne de la polymérase est une réaction en chaîne de la polymérase en temps réel.

17. Procédé selon la revendication 11, dans lequel l'un de la multiplicité des analytes d'acides non nucléiques est le PSA.

18. Procédé selon la revendication 11, dans lequel l'échantillon est un échantillon de sérum et au moins l'un de la multiplicité d'analytes d'acides non nucléiques est présent à une concentration inférieure à 10 picogrammes par millilitre.

19. Procédé selon la revendication 1, comprenant en outre :
dans l'étape (ii), la fourniture d'un complexe anticorps/analyte, dans lequel l'extrémité 5' d'un premier acide nucléique est conjuguée à l'anticorps, l'analyte comprenant un antigène reconnu par l'anticorps, et l'analyte étant lié de manière spécifique à l'anticorps par l'intermédiaire du site de combinaison d'un antigène de l'anticorps ;
dans l'étape (iii), la fourniture d'un support solide comprenant un second acide nucléique, dans lequel l'extrémité 5' du second acide nucléique est liée au support solide, dans lequel le second acide nucléique est substantiellement complémentaire du premier acide nucléique sur son extrémité 3' ;
dans l'étape (iv), la liaison du complexe anticorps/analyte au support solide au moyen d'un pont d'acide nucléique double brin, dans lequel le pont d'acide nucléique est formé par l'hybridation des extrémités non liées des premier et second acides nucléiques ; et
dans l'étape (v), l'élution du complexe anticorps/analyte à partir du support solide au moyen d'un acide nucléique de déplacement.

20. Procédé selon la revendication 1, dans lequel l'étape (v) comprend l'étape d'élution du complexe de rapporteur dépendant de l'analyte d'acide non nucléique/second récepteur qui est fixé à un support solide au moyen d'un pont d'acide nucléique par la mise en contact du complexe de rapporteur dépendant de l'analyte/second récepteur avec l'acide nucléique de déplacement dans des conditions qui favorisent le déplacement du complexe de rapporteur dépendant de l'analyte/second récepteur avec l'acide nucléique de déplacement.
